(19) Europäisches Patentamt
European Patent Office
Office européen des brevets

(11) EP 0 711 292 B1

(12) **EUROPEAN PATENT SPECIFICATION**

(45) Date of publication and mention of the grant of the patent:
**23.01.2002 Bulletin 2002/04**

(21) Application number: **94921036.3**

(22) Date of filing: **15.07.1994**

(51) Int Cl.[7]: **C07D 471/10**, A61K 31/435, C07D 498/10, C07D 453/02, C07D 497/10, C07D 513/10

(86) International application number:
**PCT/GB94/01543**

(87) International publication number:
**WO 95/03303 (02.02.1995 Gazette 1995/06)**

(54) **AZA SPIRO COMPOUNDS ACTING ON THE CHOLINERGIC SYSTEM WITH MUSCARINIC AGONIST ACTIVITY**

AUF DAS CHOLINERGE SYSTEM MIT MUSCARIN AGONISTICHER AKTIVITÄT WIRKENDE AZA-SPIRO-VERBINDUNGEN

AZA SPIRO-COMPOSES AGISSANT SUR LE SYSTEME CHOLINERGIQUE AVEC UNE ACTIVITE AGONISTE MUSCARINIQUE

(84) Designated Contracting States:
**AT BE CH DE DK ES FR GB GR IE IT LI LU NL PT SE**

(30) Priority: **20.07.1993 US 94855**

(43) Date of publication of application:
**15.05.1996 Bulletin 1996/20**

(73) Proprietors:
- **KOSMIN, Gerald, Emmanuel**
  **Kenton, Harrow HA3 0DZ (GB)**
  Designated Contracting States:
  **GB**
- **STATE OF ISRAEL, REPRESENTED BY PRIME MINISTER'S OFFICE, ISRAEL INSTITUTE FOR BIOLOGICAL RESEARCH**
  **Ness-Ziona 70450 (IL)**
  Designated Contracting States:
  **BE CH DE DK ES FR GR IE IT LI LU NL PT SE AT**

(72) Inventors:
- **FISHER, Abraham**
  **58481 Holon (IL)**
- **KARTON, Yishal**
  **70400 Ness-Ziona (IL)**
- **MARCIANO, Daniele**
  **47201 Ramat-Hasharon (IL)**
- **BARAK, Dov**
  **76502 Rehovot (IL)**
- **MESHULAM, Haim**
  **59313 Bat Yam (IL)**

(74) Representative: **Ruffles, Graham Keith**
**MARKS & CLERK, 57-60 Lincoln's Inn Fields**
**London WC2A 3LS (GB)**

(56) References cited:

| | |
|---|---|
| **EP-A- 0 189 370** | **EP-A- 0 205 247** |
| **EP-A- 0 303 391** | **EP-A- 0 311 313** |
| **EP-A- 0 314 444** | **EP-A- 0 452 101** |
| **JP-A- 2 164 882** | **JP-A- 2 247 183** |
| **JP-A- 63 208 590** | **TW-A- 201 312** |

- **J.MED.CHEM., vol.30, 1987, WASHINGTON pages 969 - 975 SAUNDERS,J. ET AL. 'Synthesis and Charactersiation of all four isomers....'**
- **J.MED.CHEM., vol.31, 1988, WASHINGTON pages 486 - 491 SAUNDERS,J. ET AL. '2-Methyl-1,3-dioaazaspiro[4.5]decanes as novel Muscarinic Cholinergic Agonsits'**
- **J.MED.CHEM., vol.35, 1992, WASHINGTON pages 1541 - 1550 NORDVALL,G. ET AL. 'Analogues of the Muscarinic Agent...'**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

FIELD AND BACKGROUND OF THE INVENTION

The present invention relates to spiro five-membered ring compounds in which the ring which is spiro-connected to the five-membered ring as set forth herein is a saturated bridged or unbridged ring containing one or two nitrogen atoms; to pharmaceutical compositions containing the spiro compounds and to a method for treating diseases of the central and peripheral nervous system using such spiro-compounds or pharmaceutical compositions.

Novel spiro-quinuclidine compounds, in which oxathiolane rings were connected in spiro manner with quinuclidine rings, were described e.g. in European Patent Application No. 0205247 A2, published December 17, 1986, and in U.S. Patents Nos. 4.855.290 (issued August 8, 1989), 4,981,858 (issued January 1, 1991). 4,900,830 (issued February 13, 1990) and 4,876,260 (issued October 24, 1989). Similarly, some spiro-oxazolines have been described in U. S. Patent No. 5,053,412 (issued October 5,053,412). while some spiro-oxazolines and some spiro-thiazolines have been described in United States Patent Application Serial No. 07/685,397. It is to be understood that the entire contents of the above-mentioned patents and of U.S.S.N. 07/685,397, as well as any other patents and literature articles mentioned in the present patent application are incorporated herein by reference. The novel compounds of the above-mentioned patents possess central nervous system activity. The biological activity of 2-methylspiro(1,3-oxathiolane-5,3')quinuclidine, which exists as geometrical cis- and transisomers depending upon whether the 2-methyl group is located on the same side of the oxathiolane ring as the quinuclidine ring nitrogen atom (cis) or on the other side of the quinuclidine ring nitrogen atom (trans), was in particular extensively investigated, and it was found on the basis of pre-clinical tests that the cis- compound (code no. AF102B) was especially promising for the control of senile dementia of Alzheimer's type (SDAT). It is also of interest that each of the cis- and trans-isomers may be optically resolved, and the biological activity of the optical isomers was also investigated in a number of cases.

Japanese Kokai Tokyo Koho JP 02,247,183 describes spiropiperidine dioxolanes and - oxathiolanes and their sulfur analogs as muscarinic m1 [1 tiefer gesetzt] agonists. GB 1,203,440 describes psychotropic heterocyclic spiro compounds.

It is a principal object of the invention to provide novel spiro-compounds and new uses for known and nord spiro-compounds. Further objects of the invention, and especially those which relate to the provision of useful pharmaceutical compositions and methods for the treatment of disease, will be apparent from the description which follows.

DESCRIPTION OF THE INVENTION

The present invention provides for the use of a compound of the formula (I) in the preparation of a medicament for use in the treatment of a disease of the central or peripheral nervous system in a mammal, the formula (I) being:

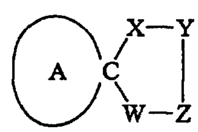

wherein X is >S, Y is >CRR', Z is >C=O and W is $>NR^o$,

in which R is selected from H, $C_{1-6}$-alkyl, $C_{1-6}$-alkoxy, $C_{2-6}$-hydroxyalkyl, $C_{2-6}$-alkenyl, $C_{2-6}$-alkynyl, phenyl and $C_{1-6}$-alkyl substituted by 1, 2 or 3 phenyls;

R' is selected from H, $C_{1-6}$-alkyl, $C_{1-6}$-alkoxy, $C_{2-6}$-hydroxyalkyl, $C_{2-6}$-alkenyl, $C_{2-6}$-alkynyl, phenyl and $C_{1-6}$-alkyl substituted by 1, 2 or 3 phenyls; and

$R^0$ is selected from H, $C_{1-6}$-alkyl, $C_{1-6}$-alkoxy, $C_{2-6}$-hydroxyalkyl, $C_{2-6}$-alkenyl, $C_{2-6}$-alkynyl, phenyl, $C_{1-6}$-alkyl substituted by 1, 2 or 3 phenyls, and $C_{1-6}$-alkanoyl; and

A is a ring (M):

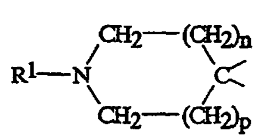

which is unsubstituted or is substituted by 1 - 3 substituents selected from $C_{1-6}$-alkyl and hydroxyl, and wherein n and p are each 0, 1, 2 or 3, provided that n + p is 1 - 3; and $R^1$ is selected from H, $C_{1-6}$-alkyl, $C_{2-6}$-alkenyl, $C_{2-6}$-alkynyl, $C_{3-7}$-cycloalkyl, $C_{1-6}$-alkyl substituted by 1 - 6 halogen atoms, hydroxy-$C_{1-6}$-alkyl, $C_{1-6}$-alkoxy, $C_{1-6}$-alkylthio, $C_{1-6}$-alkoxy-$C_{1-6}$-alkyl, carboxy-$C_{1-6}$-alkyl, ($C_{1-6}$-alkoxy)carbonyl-$C_{1-6}$-alkyl, amino-$C_{1-6}$-alkyl, mono-($C_{1-6}$-alkyl)amino-$C_{1-6}$-alkyl, di-($C_{1-6}$-alkyl)amino-$C_{1-6}$-alkyl, 2-oxo-pyrrolidin-1-yl-methyl, aryl, diarylmethylol, $C_{1-6}$-alkyl substituted by one or two aryl groups, $C_{1-6}$-alkanoyl and arylcarbonyl, where aryl denotes unsubstituted phenyl or phenyl substituted by 1 - 3 substituents selected from halogen, $C_{1-6}$-alkyl, $C_{1-6}$-alkoxy and $CF_3$;
including pharmaceutically acceptable salts, quaternary compounds structurally derived from the compounds at a tertiary nitrogen atom, enantiomers and racemates thereof.

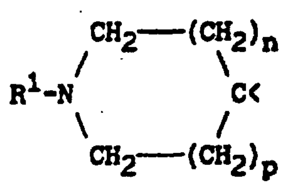

(M)

wherein the structure is unsubstituted or is substituted by 1-3 substituents selected from $C_{1-6}$ alkyl and hydroxyl, m is 1, 2 or 3, and n and p are each independently 0, 1, 2 or 3, provided that n + p = 1-3; $R^1$ is selected from hydrogen, $C_{1-6}$-alkyl, $C_{2-6}$-alkenyl, $C_{2-6}$-alkynyl, $C_{3-7}$- cycloalkyl, $C_{1-6}$-alkyl substituted by 1-6 halogen atoms, hydroxy- $C_{1-6}$-alkyl, $C_{1-6}$-alkoxy, $C_{1-6}$-alkylthio, $C_{1-6}$-alkoxy-$C_{1-6}$-alkyl, carboxy-$C_{1-6}$-alkyl, ($C_{1-6}$-alkoxy)carbonyl -$C_{1-6}$-alkyl, amino-$C_{1-6}$-alkyl, mono-($C_{1-6}$-alkyl)amino-$C_{1-6}$-alkyl, di-($C_{1-6}$-alkyl)amino-$C_{1-6}$-alkyl, 2-oxo-pyrrolidin-1-yl-methyl, aryl, diarylmethylol, $C_{1-6}$-alkyl substituted by one or two aryl groups, $C_{1-6}$-alkanoyl and arylcarbonyl; and aryl denotes unsubstituted phenyl or phenyl substituted by 1-3 substituents selected from halogen, $C_{1-6}$-alkyl, $C_{1-6}$-alkoxy and $CF_3$; and $R^2$ and $R^3$ are independently selected from $C_{1-4}$ alkyl.

Exemplary compounds of the invention are:

An enantiomer of a compound of the formula (I):

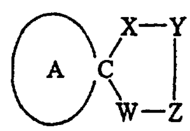

wherein X is >S, Y is >CRR', Z is >C=O and W is >NR, in which R is selected from H, $C_{1-6}$-alkyl, $C_{1-6}$-alkoxy, $C_{2-6}$-hydroxyalkyl, $C_{2-6}$-alkenyl, $C_{2-6}$-alkynyl, phenyl and $C_{1-6}$-alkyl substituted by 1, 2 or 3 phenyls; R' is selected from H, $C_{1-6}$-alkyl, $C_{1-6}$-alkoxy, $C_{2-6}$-hydroxyalkyl, $C_{2-6}$-alkenyl, $C_{2-6}$-alkynyl, phenyl and $C_{1-6}$-alkyl substituted by 1, 2 or 3 phenyls; and
$R^0$ is selected from H, $C_{1-6}$-alkyl, $C_{1-6}$-alkoxy, $C_{2-6}$-hydroxyalkyl, $C_{2-6}$-alkenyl, $C_{2-6}$-alkynyl, phenyl, $C_{1-6}$-alkyl substituted by 1, 2 or 3 phenyls, and $C_{1-6}$-alkanoyl; and
A is a ring (M):

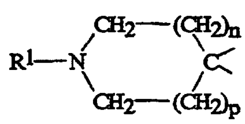

wherein n and p are each 0, 1, 2 or 3, provided that n + p is 1 - 3; and
$R^1$ is selected from H, $C_{1-6}$-alkyl, $C_{2-6}$-alkenyl, $C_{2-6}$-alkynyl, $C_{3-7}$-cycloalkyl, $C_{1-6}$-alkyl substituted by 1 - 6 halogen atoms, hydroxy-$C_{1-6}$-alkyl, $C_{1-6}$-alkoxy, $C_{1-6}$-alkylthio, $C_{1-6}$-alkoxy-$C_{1-6}$-alkyl, carboxy-$C_{1-6}$-alkyl, ($C_{1-6}$-alkoxy)carbonyl-$C_{1-6}$-alkyl, amino-$C_{1-6}$-alkyl, mono-($C_{1-6}$-alkyl)amino-$C_{1-6}$-alkyl, di-($C_{1-6}$-alkyl)amino-$C_{1-6}$-alkyl, 2-oxo-pyrrolidin-1-yl-methyl, aryl, diarylmethylol, $C_{1-6}$-alkyl substituted by one or two aryl groups, $C_{1-6}$-alkanoyl and arylcarbonyl, where aryl denotes unsubstituted phenyl or phenyl substituted by 1 - 3 substituents selected from halogen, $C_{1-6}$-alkyl, $C_{1-6}$-alkoxy and $CF_3$; including pharmaceutically acceptable salts and quaternary compounds structurally derived from the

compounds at a tertiary nitrogen atom;

An enantiomer which is AF267B, that is (-)AF267, being a compound of formula (K):

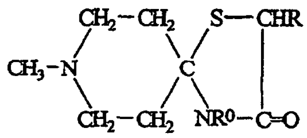

where R is ethyl and $R^0$ is hydrogen.

A compound AF267 which is a compound of formula (K):

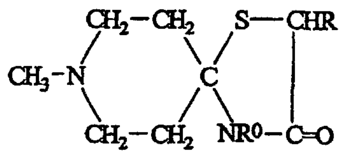

where R is ethyl and $R^0$ is hydrogen;

The hydrochloride salt of AF267.

A compound AF266 which is a compound of formula (K):

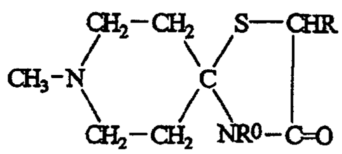

where R is methyl and R° is methyl;

A compound AF272 which is a compound of formula (K):

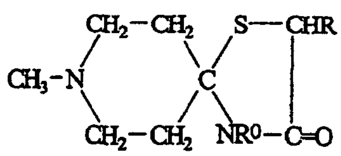

where R is ethyl and R° is methyl;

A compound AF261 which is a compound of formula (K):

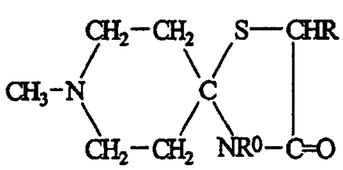

where R is methyl and $R^0$ is hydrogen.

The present invention moreover provides a pharmaceutical composition for use in treating diseases of the central and peripheral nervous system in mammals, which comprises an amount effective for use in treating said diseases, of at least one compound having the formulae I as defined above, including their pharmaceutically acceptable salts, enantiomers and racemates, together with at least one pharmaceutically acceptable diluent, carrier or adjuvant. Such composition is preferably in a form suitable for oral, rectal, parenteral or transdermal administration (in this case the composition may comprise additionally a low molecular weight fatty acid), or for administration by insufflation or nasal spray, and may be in unit dosage form. The at least one compound of the invention as defined above, may be present in the unit dosage in an amount in the range of, e.g. about 0.5 to about 100 mg, preferably about 5 to about 100 mg, more preferably about 10 to about 50 mg.

According to a particular embodiment of the invention, the pharmaceutical composition as described in the preceding paragraph may comprise additionally at least one further pharmacologically active compound selected from physostigmine, tetrahydmaminoacridine, choline, lecithin, piracetam. aniracetam, pramiracetam, oxiracetam, 4-aminopyridine, 3,4-diaminopyridine, somatostatin, pirenzepine, N-methylatropine, N-butylscopolamine, scopolamine, clonidine, quanfamicine, propantheline, methantheline, glycopyrrolate, tropenzilium, nortriptyline, amitriptyline, imipramine, minaprine, secoverine, AFDX-116, nicotine, alaproclate, zimelidine, deprenyl and Nerve Growth Factor.

Diseases of the central or peripheral nervous system in mammals may be treated by a method which comprises administering thereto an amount effective for use in treating said diseases, of at least one compound having one of the formulae I as defined above, including their pharmaceutically acceptable salts, enantiomers, tautomers and racemates. Such compounds may of course be utilized for this purpose in the form of a pharmaceutical composition according to the invention, as defined above.

The invention also provides, in a particular embodiment, compounds of the formulae I as defined herein, having the molecular dimensions indicated below, where: r is a reference point defined by the position of an anion corresponding to the cationic form of a non-doubly-bonded nitrogen atom, defined as N*. of ring A (or A') in such compound in its most stable conformation, X* defines a ring hetero atom in the depicted 5-membered ring having any of the formulae I through IX, or X through XIII, such ring hetero atom being in a position adjacent to the spiro carbon atom, Z* defines the next but one ring atom from X* in the depicted 5-membered ring, and Q* defines the terminal C or N atom of a side-chain attached to the ring atom between atoms X* and Z* in the 5-membered ring depicted infra, side-chain hydrogen atoms being ignored for this purpose; such molecular dimensions having substantially the following values, namely: a dihedral angle r-X*-Q*-Z* = from -54° to -170°; and molecular distances r-N* = 3.0 angstroms (the reference distance), r-X* = from 5.7 to 6.75 angstroms, r-Q* = from 7.9 to 8.90 angstroms, x-Q* = from 2.4 to 2.8 angstroms; such compounds having the thus-defined molecular dimensions being characterized by having muscarinic agonist activity. This definition based on molecular dimensions is supported by biological tests, see especially Table 1, below.

In another embodiment, the invention provides a pharmaceutical composition which comprises at least one compound of the formula I as defined herein, and additionally Nerve Growth Factor (NGF), the compound(s) of the invention being present in an amount which promotes the nerve growth activity of the NGF. Most of the present compounds, unlike certain known compounds possessing central or peripheral nervous system activity. do not per se promote nerve growth activity in absence of NGF, thus allowing better control, when nerve growth promotion is desired in therapy. However, some of the most active of the present compounds promote nerve growth activity independently of NGF. Moreover, compounds of the invention which usually have activity selected from muscarinic agonist activity, amyloid precursor protein (APP) secreting activity and β-amyloids decreasing activity, and activity increasing the proportion of dephosphorylated τ proteins. Tests carried out to ascertain the biological activity of the present compounds are detailed infra.

The invention moreover provides compounds of formula (I) as depicted above, including pharmaceutically acceptable salts, quaternary compounds which are structurally derived from said compounds having a tertiary nitrogen atom, enantiomers and racemates thereof, for use in the manufacture of a medicament having biological activity selected from (a) muscarinic agonist activity, (b) neurotrophic-like or synergistic activity with NGF, (c) amyloid precursor protein (APP) secreting activity and β-amyloids decreasing activity, (d) activity increasing the proportion of dephosphorylated τ proteins, and (e) NGF-like activity. Exemplary such compounds are: 1-methylpiperidine-4-spiro-5'-(2'-methyl-1',4'-thiazolidine-3'-one); 1-methylpiperidine-4-spiro-5'-(2',4'-di-methyl-1',4'-thiazolidine-3'-one); 1-methylpiperidine-4-spiro-5'-(2'-ethyl-1',4'-thiazolidine-3'-one); 1-methylpiperidine-4-spiro-5'-(2'-ethyl-4-methyl-1',4'-thiazolidine-3'-one); piperidine-4-spiro-5'-(2'-methyl-1',4'-thiazolidine-3'-one); 1-methylpiperidine-4-spiro-5'-(2'-methyl-1',4'-thiazolidine-3'-one); 1-methylpiperidine-4-spiro-5'-(2'-ethyl-1',4'-thiazolidine-3'-one); 1-methylpiperidine-4-spiro-5'-(2'-methyl-1',4'-thiazolidine -3'-thione); and the enantiomers d- and 1-1-methylpiperidine-4-spiro-5'-(2'-methyl-1',4'-thiazolidine-3'-one) and d- and 1-1-methylpiperidine-4-spiro-5'-(2'-ethyl-1',4'-thiazolidine-3'-one).

Such compounds of the invention may thus be used for treating diseases in mammals, diagnosed as being amenable to treatment with an effective amount (in the context of the above activities a-e) of the compound(s), and

may be utilized in the form of pharmaceutical compositions comprising an effective amount of such compound(s), but are otherwise as described above.

The methods used for preparing the present compounds are essentially those known to organic chemists for the formation of the five-membered rings, ring-substitution, changing the degree of ring saturation/unsaturation, interconversion of salts and bases, quaternary salt formation, etc. Thus, while exemplary methods of preparing certain compounds of the invention will be described, other methods can also be applied to their preparation, as will be evident to the skilled person.

Thiazolidines of the invention such as structure (K) (such as AF261, R=Me. R°=H; AF267. R=Et, R°=H; AF266, R=R°=Me) may be made, e.g., as described below.

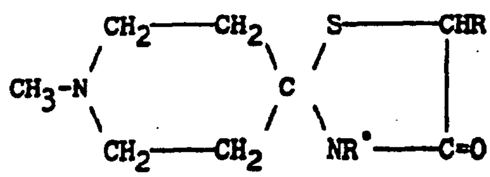

(K)

Illustratively, compounds (K) are prepared when N-methylpiperidone is reacted with $HOCHRCHR'NH_2$. or with ($HSCHRCO_2H$ + $R°NH_2$, respectively.

The spiro-compounds of the invention are in general potentially useful for the treatment of presenile and senile dementia, senile dementia of Alzheimer's type (SDAT), atypical Alzheimer's disease (Perry et al, Advances in Neurology, eds. R.J. Wurtman et al., 51:41, 1990), combined multiinfract dementia and Alzheimer's disease, age-associated memory impairments (AAMI). acute confusion disorders, emotional and attention disorders, mania, tardive-dyskinesia, hyperkinesia, mixed Alzheimer's and Parkinson's disease, aphasia, hallucinatoryparanoid states, post encephalitic amnesic syndrome, alcohol withdrawal symptoms, Huntington's chorea, Pick's disease. Friedrick's ataxia, Gilles de-la Tourette disease and Down syndrome, because all of these disease states are disturbances in which a central cholinergic hypofunction has been implicated at least to a certain extent. The present compounds are moreover potentially useful for the treatment of progressive supranuclear palsy; they are also potentially analgesic agents and thus may be useful in the treatment of severe painful conditions such as rheumatism, arthritis and terminal illness.

As indicated briefly above, the spiro-compounds of the present invention may be used in combination with at least one additional pharmacologically active compound, for example, acetylcholinesterase inhibitors such as physostigmine or tetrahydroaminoacridine; in combination with acetylcholine precursors such as choline or lecithin; in addition to "nootropic" drugs such as piracetam, aniracetam, oxiracetam, or pramiracetam: in addition to compounds that interact with $Ca_{2+}$ channels such as 4-aminopyridine or 3,4-diaminopyridine; or in addition to peptides that can have modulatory effects on acetylcholine release, such as somatostatin; in combination with a peripheral antimuscarinic agent (such as pirenzepine, N-methylatropine, N-butylscopolamine, propantheline, methantheline, glycopyrrolate, or tropenzilium) to counteract peripheral adverse effects that might be expected at high doses, such as salivation, diarrhea, gastric secretion or vomiting, or in combination with transdermal scopolamine such as $Scopoderm_{(R)}$ to counteract nausea and/or vomiting; in combination with antidepressants such as nortriptyline, amitriptyline, imipramine, minaprine in order to alleviate both the cognitive impairments and depressive symptoms associated sometimes with SDAT, AAMI, mixed SDAT/Parkinson's disease (PD); in combination with M2-antimuscarinic drugs such as secoverine, AFDX-116 (c.f. Hammer et al, 1986 Life Sci. 38:1653) in order to counteract peripheral adverse side effects that might be expected at high doses of the compounds, to counteract inhibitory effects of such agonists at central inhibitory presynaptic and postsynaptic receptors of M2 type and to potentiate the release of acetylcholine via inhibition of inhibitory autoreceptors of M2 type at intact terminals; in combination with nicotinic agonists such as nicotine in order to stimulate both the nicotinic and muscarinic receptors in the brain; in combination with an adrenergic agonist (clonidine or quanfamicine) in order to alleviate both the cognitive and other impairments associated with a mixed cholinergic-noradrenergic deficiency in SDAT; in combination with inhibitors of neuronal serotonin reuptake such as alaproclate, zimelidine in order to alleviate both the cognitive and other emotional functions in SDAT; in combination with monoamine oxidase-B inhibitors like deprenyl in order to alleviate both cognitive and other motor impairments associated with mixed states such as SDAT/PD; in combination with Nerve Growth Factor (NGF, which is administered either by a nasal spray or intracerebroventricularly).

The spiro-compounds of the present invention, with or without the aforementioned additional active substances, can be administered for example, by way of injection in a suitable diluent or carrier, per os, rectally in the form of suppositories, by way of insufflation or nasal spray, by infusion or transdermally in a suitable vehicle with or without physostigmine or tetrahydroamincacridine.

The present spiro-compounds may also be of potential use for the treatment of disorders requiring the application of a long-lasting cholinergic agent of mild local activity. Such an agent is needed in disorders such as glaucoma, as the compound is not destroyed by the enzyme which deactivates acetylcholine, i.e. acetyl- and butyryl-cholinesterase, and may also be used for the treatment of peripheral cholinergic disorders such as myasthenia gravis, urinary bladder dysfunctions, Adi's disease and Eaton-Lambert disease. These compounds might also be used in disturbances where cholinergic underactivity is induced by drugs.

Where the present spiro-compounds are anticholinergic agents (which may readily determined by the skilled person) they may potentially be used for treatment of disorders due to a cholinergic hyperfunction, whether this be spontaneous or drug-induced. Moreover, the present compounds are of potential use in the treatment of various diseases such as PD, pseudo-PD, mixed AD/PD, primary dystonias, spasmodic torticollis, cranial dystonia, depression, motion sickness, akathisia (after neuroleptic withdrawal), central hypertension, human head injury, mixed tardive dyskinesia and PD, manic-depression, as adjuncts in surgery instead of atropine, scopolamine, etc., in intoxication due to an excess of acetylcholine like inhibition of acetylcholinesterase. These may also be used in ophthalmology when either prolonged or short-term mydriasis is required.

The present spiro-compounds may also potentially be used in the treatment of disease characterized by excess peripheral-like activity such as asthma, chronic obstructive pulmonary disease, peptic ulcer disease. For these peripheral disorders it is particularly recommended to use quaternary salts of the present compounds.

Quaternary ammonium salts are widely used in therapy at the present time. Thus, examples of such cholinergic agonists are acetylcholine chloride, bethanechol chloride and carbachol (see e.g. Goodman & Gilman's "The Pharmacological Basis of Therapeutics", Seventh Edition, Macmillan Publishing Co., 1985, at page 104). Quaternary anticholinesterase agents are, e.g., neostigmine bromide, ambenonium chloride, pyridostigmine bromide, edrophonium chloride, demecarium bromide, and echothiphate iodide; pralidoxime chloride is used as a cholinesterase reactivator (see Goodman & Gilman, loc cit, at pages 122-123).

Quaternary derivatives of belladonna alkaloids, e.g. methscopolamine bromide and homatropine methylbromide, and synthetic quaternary compounds, e.g. methantheline bromide, and propantheline bromide, are used in treating gastrointestinal disorders (see Goodman & Gilman, loc cit, at pages 139-140).

In "Medicinal Chemistry" by Alfred Burger, Second Edition, Interscience Publishers, 1960, at page 497, there is mentioned a prediction that quaternary ammonium ions, regardless of their chemical structure, produce curareform paralysis and the later corroboration of this prediction. This property of quaternary compounds is utilized in anesthesia, e.g. as an adjuvant in surgical anesthesia to obtain relaxation of skeletal muscle (see Goodman & Gilman, loc cit. in Chapter 11 under the title of "Neuromuscular Blocking Agents", at pages 222-235).

This neuromuscular blocking activity of quaternary compounds. as discussed above, has not prevented development and the clinical application of quaternary compounds in therapeutics in the ensuing years. The skilled person would be aware that many factors influence the selection of any compound (including a quaternary compound) for application in clinical therapy, e.g., effectiveness for the intended purpose, safety, possible side-effects and therapeutic index. The skilled addressee would thus well understand how to interpret the expression "pharmaceutically acceptable quaternary compounds", which are structurally derived from the inventive compounds having a tertiary nitrogen atom, as this expression is used in the present specification and claims, in the light of the relevant knowledge available in the art.

The invention will now be illustrated by the following Examples.

Example 1: 1-Methylpiperidine-4-spiro-5'-(2'-methyl-1',4'-thiazolidine-3'-one) AF261; and Example 2: 1-Methylpiperidine-4-spiro-5'-(2',4'-dimethyl-1',4'-thiazolidine-3'-one) AF266.

a) A mixture of 1-methyl-4-piperidone (5.65 g, 0.050 mole), thiolactic acid (6.37 g, 0.060 mole) and ammonium carbonate (7.20 g) in benzene (150 ml) was heated under reflux for 16h, when a solid sublimed (ammonium carbonate) which condensed outside the reaction flask. From time to time this solid was added again to the reaction flask together with an additional portion of new ammonium carbonate (total 6.2 g). Finally, the benzene was removed by distillation and the residue washed with ether to give an ether soluble material (0.76 g) and an insoluble one (12.9 g), which was dissolved in a small amount of methanol and made basic with a solution of ammonia in methanol. It was evaporated and chromatographed on a column of silica gel (Merck 60). Elution with a solvent mixture of chloroform/methanol/ ammonia 89:10:1 gave a fraction (0.61 g) which was identified as 1-methylpiperidine-4-spiro-5'-(2',4'-dimethyl-1',4'-thiazolidine-3'-one) AF266. It is a low melting point hygroscopic solid. $^1$H-NMR ($CDCl_3$) $\delta$ 1.53(d, j=7Hz, $CH_3CH$-); 1.69(m, 2H); 2.16-2.44(m, 4H); 2.32(s, $CH_3N$-); 2.87(m, 2H); 2.90(s, $CH_3NCO$-); 3.79(q, j=7Hz, $CH_3CH$-) ppm. $^{13}$C-NMR ($CDCl_3$) $\delta$ 19.7($CH_3CH$-); 27.2($CH_3NCO$-); 36.0 and 37.3(C6&C10); 39.7 (C2); 45.3($CH_3N$-); 51.8 and 52.2 (C7&C9); 68.5(C5); 172.8(C3) ppm.

The hydrochloric acid salt of AF266 was obtained by treating a solution of the free base in ether with a solution of hydrochloric acid in isopropyl alcohol to give a hygroscopic solid which was crystallized from isopropyl alcohol m. p. 238-240°C (dec.). $^1$H-NMR ($D_2O$, HCl salt) 6 1.48(d, j=7.2Hz, $CH_3CH$-); 2.05(m, 2H); 2.57(m, 2H); 2.89 and 2.91(2s, $CH_3N^+$- and $CH_3NCO$-); 3.31(m, 2H); 3.61(m, 2H); 4.04(q, j=7.2Hz, $CH_3CH$-) ppm. MS m/e 214($M^+$); 181; 156; 125; 124; 96; 71; 70; 57(100%); 43.

Continuing the elution of the column with a solvent mixture of chloroform/methanol/ammonia 85:14:1 gave a fraction (3.50 g) which was crystallized from ether-dichloromethane to give 1-methylpiperidine-4-spiro-5'-(2'-methyl-1',4'-thiazolidine-3'-one) AF261 as needles (1.83 g) m.p. 133-134°C.

$^1$H-NMR ($CDCl_3$) $\delta$ 1.53(d, j=7Hz, $CH_3CH$-); 1.9-2.20(m, 4H); 2.20-2.42(m, 2H); 2.30(s, $CH_3N$-); 2.68(m, 2H); 3.85 (q, j=7Hz, $CH_3CH$-); 6.90(bs, -NH-) ppm.

$^{13}$C-NMR ($CDCl_3$) $\delta$ 19.9($CH_3CH$-); 41.0 and 41.3(C6&C10); 41.3(C2); 45.7($CH_3N$-); 52.5 and 52.8 (C7&C9); 63.6 (C5); 176.3(C3) ppm. MS m/e 200($M^+$); 167. 140($M^+$-$CH_3CHS$-); 91; 71; 70; 69; 57.

The hydrochloric acid salt of AF261 was obtained by treating a solution of the free base in ether with a solution of hydrochloric acid in isopropyl alcohol, m.p. 285-287°C.

$^1$H-NMR (HCl salt, in $D_2O$) $\delta$ 1.50( d. j=7.1Hz, $CH_3CH$-); 2.17-2.44(m, 4H); 2.89( s, $CH_3N$-); 3.24( m, 2H); 3.59( m, 2H); 4.10( q, j=7.1Hz, $CH_3CH$-) ppm.

b) A mixture of 1-methyl-4-piperidone (5.65 g, 0.050 mole). thiolactic acid (6.37 g, 0.060 mole) and ammonium carbonate (7.20 g) in benzene (100 ml) was sealed in a stainless steel pressure vessel and heated overnight at 95°C with magnetic stirring. The solvent was removed from the reaction mixture and the residue dissolved in a small amount of methanol and loaded on a dry column of silica gel (Merck 60, 200 g). Elution with a solvent mixture of chloroform/methanol/ammonia(aq.) 94:5:1 gave first AF266 (0.71 g) and afterwards AF261 (3.02 g).

c) A mixture of 1-methyl-4-piperidone (2.50 g. 22.1 mmole), thiolactic acid (2.81 g, 26.5 mmole) and methylamine (0.90 g, 29.0 mmole) in toluene (60 ml) was sealed in a stainless steel pressure vessel and heated at 96°C with stirring for 24h. The reaction mixture is a viscous oil from which separated a precipitate and a toluene solution. The solution was separated and the solvent removed by distillation. The residue (1.68 g) was distilled at reduced pressure to give pure 1-methylpiperidine-4-spiro-5'-(2',4'dimethyl-1',4'-thiazolidine-3'-one) AF266 b.p.100°C (0.35 mm Hg) as a solid (1.02 g) m.p. 43-45°C.

Example 3: 1-Methylpiperidine-4-spiro-5'-(2'-ethyl-1',4'-thiazolidine-3'-one) AF267; and Example 4: 1-Methylpiperidine-4-spiro-5'-(2'-ethyl-4-methyl-1',4'-thiazolidine-3'-one) AF272.

The title compound AF267 was prepared as described for the preparation of AF261, using 2-mercaptobutyric acid instead 2-mercaptopropionic acid (thiolactic acid). It was crystallized from acetone m.p. 140-141°C.

$^1$H-NMR ($CDCl_3$) $\delta$ 1.02(t, j=7.2Hz, $CH_3CH_2$-); 1.75 and 2.06(m, $CH_3CH_2$-); 1.98(m, 4H); 2.30(s, $CH_3N$-); 2.32(m, 2H); 2.67(m, 2H); 3.81(dd, -CHS-); 6.35(bs, -NH-) ppm.

$^{13}$C-NMR ($CDCl_3$) $\delta$ 11.4($CH_3CH_2$-); 27.1($CH_3CH_2$-); 41.5(C6&C10);
45.8($CH_3N$-); 49.0(C2); 52.7 and 52.9(C7&C9); 63.7(C5); 175.5(C3) ppm.
MS m/e 214($M^+$); 181; 140; 71; 57.

The hydrochloric acid salt of AF267 had m.p. 267-269°C (dec.).

As a by product 1-Methylpiperidine-4-spiro-5'-(2'-ethyl-4'-methyl-1',4'-thiazolidine-3'-one)AF272 was obtained as a viscous oil.

$^1$H-NMR ($CDCl_3$) $\delta$ 1.00(t, j=7.4Hz, $CH_3CH$-); 1.67(m, 2H); 1.58-1.78(m) and 2.13(m) ($CH_3CH_2$-); 2.20-2.42(m,4H); 2.32(s, $CH_3N$);
2.89(s, $CH_3NCO$-); 2.90(m, 2H); 3.76(dd, j1=3.6Hz, j2=9.0Hz, $CH_3CH_2CH$-) ppm.
MS m/e 228($M^+$); 195; 170; 138; 125; 96; 71; 70; 57. ml acetic acid and 0.5 ml aqueous hydrogen peroxide (32%) was stirred at 25°C for twenty minutes. The reaction mixture was acidified by addition of excess of hydrochloric acid and diluted with 20 ml petroleum-ether/ether 4/1. The precipitate was dissolved in aqueous sodium carbonate and the solution was extracted with chloroform. The chloroform extract was dried, evaporated and the resultant mass was chromatographed on a preparative silica-gel plate (chloroform/methanol/aqueous-ammonia 80/20/1) to yield 3.3 mg of oil, which was precipitated from ether by oxalic acid to yield one isomer (>90% purity) of a crystalline solid.

$^1$H-NMR ($CDCl_3$,free base) $\delta$ 1.52(d, 3H,J=6Hz))$CH_3CH$-; 1.85-2.7[m, 8H]; 2.36(s,3H)$CH_3$-N; 3.55(q,J=6Hz)$CH_3CH$-); 7,0(bs,1H) ppm.
MS m/e 216($M^+$); 199; 167; 149; 125; 111.

Example 5: Piperidine-4-spiro-5'-(2'-methyl-1',4'-thiazolidine-3'-one) AF263

A mixture of 1-methylpiperidine-4-spiro-5'-(2'-methyl-1',4'-thiazolidine-3'-one) (AF261) (250 mg, 1.25 mmole) and $\alpha$-chloroethyl chloroformate (0.15 ml, 1.4 mmole) was heated in 5 ml dichloroethane at 60°C for ninety minutes.

The dichloroethane was removed under a stream of nitrogen and the oily residue was dissolved in methanol and heated at 60°C for one hour. The methanol was evaporated, the residue was dissolved in aqueous sodium carbonate, extracted with chloroform and separated on a preparative silica-gel plate (chloroform/methanol/aqueous ammonia 80/20/1) to yield 20 mg free base that was precipitated as a crystalline oxalic acid salt.

$^1$H-NMR ($D_2O$, oxalic acid salt) δ 1.51(d, 3H,J=6Hz, $CH_3CH$-); 2.2-2.35 (m, 4H); 3.1-3.6(m,4H); 4.1(q,J=6Hz,$CH_3CH$-) ppm.

MS m/e 186($M^+$); 153; 126; 57.

Example 6: 1-Methylpiperidine-4-spiro-5'-(2'-methyl-1',4'-thiazolidine-3'-one) AF261, and its optical isomers (+)AF261 & (-)AF261

Compound AF261 as a free base (3.23g., 16 mmole) and di-p-toluoyl-D-tartaric acid (5.8 g, 15 mmole) were placed in 250 ml flask, isoamyl alcohol 28 g and toluene 130 g were added and the mixture was refluxed for 5 minutes. The clear solution was partially evaporated under reflux by a stream of $N_2$ until the formation of a slight turbidity and allowed to stand overnight. The resulting precipitate was filtered off and recrystallized from isoamyl alcohol-toluene several times until enantiomeric purity of above 98% (by GC and NMR) was obtained. The solid product was basified and precipitated as the hydrochloric acid salt to yield 207 mg white non-hygroscopic solid $[\alpha]_D^{25}$ = -54.8° (hydrochloric acid salt in methanol).

The mother liquor was evaporated, basified and treated with Di-p-toluoyl-L-tartaric acid and crystallized four times until obtaining enantiomeric purity of above 98% (by GC and NMR). The solid product was basified and precipitated as the hydrochloric acid salt to yield 207 mg white non-hygroscopic solid $[\alpha]_D^{25}$ = +55.7° (hydrochloric acid salt in methanol).

Determination of optical purity was done by NMR using (R)-(-)-2.2.2-trifluoro-1-(9-anthryl)ethanol as resolving agent. 7.5 mg of it vs 2.5 mg of each enantiomer in C6D6 shows the presence of a single enantiomer. The NMR spectrum of the hydrochloric acid salt was identical to that of the (±) AF261.

The purity of each enantiomer was determined by GC using a capillary column Chrompack XE-60-S-Val-S-α-PEA. WCOT fused silica 50m., id 0.26 mm., od 0.35 mm., flow 0.9 ml $N_2$, oven temperature 175°C; retention time 64 min. and 65 min. The NMR spectrum of the hydrochloric acid salt was identical to that of the (±) AF269.

Example 7: 1-Methylpiperidine-4-spiro-5'-(2'-ethyl-1',4'-thiazolidine-3'-one) AF267, and its optical isomers (+)AF267 & (-)AF267

Compound AF267 as a free base and di-p-toluoyl-D-tartaric acid were treated according to the procedure developed for AF261 (see example 6), until enantioneric purity of above 90% (by GC ) was obtained. The solid product was basified and precipitated as the HCl salt to yield 207 mg white non-hygroscopic solid, $[\alpha]_D^{25}$ = -67.0° (HCl salt in methanol, >90% optical purity). The mother liquor was evaporated, basified and treated with di-p-toluoyl-L-tartaric acid in the same manner. After four recrystallizations, the resulting product (>90%ee) was basified and precipitated as the HCl salt, $[\alpha]_D^{25}$ = +70.8* (HCl salt in methanol, >95% optical purity).

The purity of each enantiomer was determined by GC using a capillary column Chrompack XE-60-S-Val-S-α-PEA, WCOT fused silica 50m., id 0.26 mm., od 0.35 mm., flow 0.9 ml N2. oven temperature 175°C; retention time 83 min. and 88 min. The NMR spectrum of the hydrochloric acid salt was identical to that of the (+) AF267.

The compounds according to the invention exhibit pharmacological activity and are therefore useful as pharmaceuticals, e.g. for therapy. More particularly, the spiro-compounds provided by the present invention have central and peripheral (or both) activity on the nervous system. One common characteristic activity is on the cholinergic system where the compounds are ligands (e.g. agonists or antagonists) on the muscarinic receptors.

The agonistic or antagonistic profile of the compounds was evaluated in a number of tests including computer-assisted molecular evaluation, biological testing in vitro and in vivo. Computer-assisted molecular evaluation

For muscarinic agonistic activity a pharmacophoric model, based on examination of numerous agonists, was constructed. The model includes definition of those parts, in the structure of the agonists that are essential for activity, their mutual spacial orientation and to some extent the maximal volume allowed for a ligand to be an agonist.

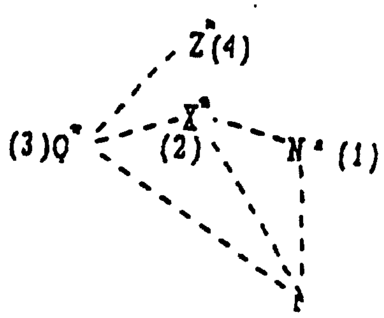

General

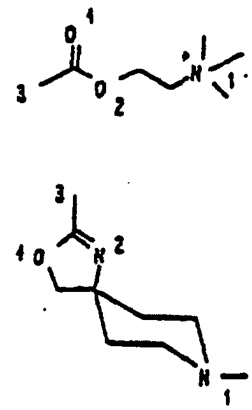

Examples

In the above formulae, point r is a negative charge interacting with the cationic head of the agonist. Its position relative to the nitrogen is defined in the model. Important distances (D) are as follows: D(r-N*) = 3.0; D(r-X*) = 6.50; D (r-Q*) = 8.70; D(x*-Q*) = 2.45. The optimal dihedral angle r-x*-Q*-z* = -85. Deviation from those optimal model parameters within certain limits does not abolish activity (Table 1).

Certain important features of this model are listed below:

1. The model allows one to distinguish between full and partial muscarinic agonists. The distinction is based upon correlation between the distance r-X* and agonistic efficacy.
2. The nature of the atom in position 4, corresponding to the carbonyl oxygen in acetylcholine can vary considerably as well as the dihedral angle r-x*-Q*-z* within the class of muscarinic agonists.
3. When the distance r-Q* becomes too large for agonistic activity, weakly binding antagonists are obtained.

From this model prediction regarding receptor subtype specificity of a muscarinic agonist can be made, based upon the agonist structural rigidity; the nature of Z* and the model parameters.

The pharmacophoric model can be used in screening of new compounds for potential muscarinic activity in the following way a: the new structure is optimized to determine its low energy conformers; b: these conformers are examined for the proper arrangement of the pharmacophoric elements. Alternatively the new structure can be forced into the pharmacophoric conformation by using one of the induced fit routines. The resulting conformation is then compared to the low energy conformation of the same structure. Application of these procedures produced a reliable answer in all the cases examined (Table 1). Albeit there is no way to predict whether muscarinic activity is manifested only by compounds conforming to this model, those that do fit are active as agonists.

The limitation of volume for the cationic head for muscarinic agonists is inferred from docking experiments of the relevant structures (as depicted in Table 1) into a molecular model of the transmembrane domain of the ml muscarinic receptor. Compounds larger than quinuclidine derivatives cannot be accommodated by the macromolecular binding site.

The compounds listed in Table 1 are divided into four groups. The first group includes several well known muscarinic agonists. Their structures are characterized by optimal pharmacophoric parameters. The second group includes agonists that display suboptimal pharmacophoric patterns and are therefore mainly partial agonists. Structures of the compounds in the third group deviate from the pharmacophoric pattern for muscarinic activity, defined by the previous groups. These compounds are therefore either antagonists or devoid of muscarinic activity. Group four lists some of the potential muscarinic agonists based on their pharmacophoric parameters. 2-ethyl-8-methyl-1-thia-4,8-diaza-spiro[4.5]decan-3-one (AF267); 2,8-dimethyl-1-thia-4,8-diaza-spiro[4.5]decan-3-one (AF261);

Table 1:

| Pharmacophoric parameters for muscarinic agonists. | | | | | |
|---|---|---|---|---|---|
| Agonist | Selectivity | r-X* (A) | r-Q* (A) | X*-Q* (A) | r-X*-Q*-Z* |
| (Group 1) | | | | | |

Table 1: (continued)

| Pharmacophoric parameters for muscarinic agonists. | | | | | |
|---|---|---|---|---|---|
| Agonist | Selectivity | r-X* (A) | r-Q* (A) | X*-Q* (A) | r-X*-Q*-Z* |
| ACh | M2>M1 | 6.40 | 8.44 | 2.40 | -86 |
| Dioxolane | M2>M1 | 6.51 | 8.71 | 2.42 | -85 |
| Muscarine | M2>M1 | 6.50 | 8.53 | 2.44 | -75 |
| Methylfurm♦ | M1>M2 | 6.40 | 8.54 | 2.43 | -59 |
| Oxatholane | M1>M2 | 6.55 | 8.90 | 2.43 | -25 |
| (Group 4) | | | | | - |
| AF261 | | 5.91 | 7.80 | 2.37 | -107 |

♦methylfurmethide

Biological Testing

**Test No. 1.** Binding to muscarinic receptors in the brain; competition with [$^3$H]QNB, [$^3$H]NMS and [$^3$H]OXO-M in membranes prepared from rat cortex and cerebellum.

Rat cerebral cortex and cerebellar membrane preparations, using the ligands [$^3$H]NMS, [$^3$H]Pirenzepine and [$^3$H]oxotremorine-M were used for evaluating the new compounds (Table 2).

**Table 2: Competition of tested compounds with [$^3$H]PZ, [$^3$H]QNB or [$^3$H]NMS (rat cortex), and [$^3$H]QNB or [$^3$H]NMS (cerebellum), respectively.**

**Cortex**

| Compound | [$^3$H]PZ | | [$^3$H]QNB | | [$^3$H]NMS | |
|---|---|---|---|---|---|---|
| | $K_H$ μM(%) | $K_L$ μM | $K_H$ μM(%) | $K_L$ μM | $K_H$ μM(%) | $K_L$ μM |
| Carbachol | 0.06(38) | 18.6 | 6.8 (18) | 980 | 0.1 (41) | 11 |
| Oxotremorine | | | | 2.4 | | |
| McN-A-343 | | | | 7.9 | | |

Table 2: (continuation)

**Cortex**

| Compound | $[^3H]PZ$ | | $[^3H]NMS$ | |
|---|---|---|---|---|
| | $K_H$ µM(%) | $K_L$ µM | $K_H$ µM(%) | $K_L$ µM |
| AF261 | 0.03(26) | 25 | 8.6 (33) | 100 |
| AF261A | | 18 | | 20 |
| AF261B | 0.13(34) | 6.7 | 0.032(31) | 4.2 |
| AF263 | 0.33(14) | 10 | 0.19(23) | 13 |
| AF267* | | 2.8* | | 5.88 |
| AF267A | | | | 10.9 |
| AF267B | | | | 4.3 |

*Following $Cu^{2+}$ treatment, the competition assay with $[^3H]PZ$ exposed two-site binding sites for AF267 ($K_H$ = 22nM (18%); $K_L$ = 2.5 µM). This observation indicates that AF267 binds to muscarinic receptors in rat cerebral cortex as an agonist (Fisher et al. J. Pharmacol. Exptl. Therap. 257: 392-403 (1991).

**Table 2: (continuation)** **Cerebellum**

| Compound | $[^3H]$QNB | | $[^3H]$NMS | |
|---|---|---|---|---|
| | $K_H$ µM(%) | $K_L$ µM | $K_H$ µM(%) | $K_L$ µM |
| Carbachol<br>Oxotremorine<br>McN-A-343 | 1.5 (54) | 52<br>0.8<br>24.4 | 0.02(56) | 6 |
| AF261 | | | 0.83(45) | 23 |
| AF261A | | | | 18 |
| AF261B | | | 0.29(53) | 24 |
| AF267 | | | | 2.3 |
| AF267A | | | | 6.9 |
| AF267B | | | 0.15(26) | 3.2 |

The calculated ratios of $Ki^{PZ}/Ki^{NMS}$ or $Ki^{PZ}/Ki^{QNB}$ (Table 2) are a commonly used indication for M1 selectivity of muscarinic ligands, lower ratios being indicative of better M1 selectivity. In addition the ratios of $Ki^{PZ}/Ki^{QNB}$ from rat cerebral cortex vs cerebellar membrane preparations are also indicative of M1 selectivity of muscarinic ligands, lower ratios than 1 being indicative of M1 selectivity (Fisher et al, J. Pharmacol. Exptl. Therap. 257: 392-403 (1991). Using these assays we could detect that some of the compounds in this invention show a relatively high preference for M1 muscarinic receptors. Such are: AF261, AF267.

The $[^3H]$NMS binding curves of some of the new compounds in the cerebral cortex membranes indicated interaction with two sites: about a quarter of the binding sites exhibited high affinity towards these compounds. This may indicate high agonistic efficacy in this preparation. Such compounds are: AF261, AF263 .

Surprisingly, one of the new compounds exhibited two-site competition curves with $[^3H]$PZ in rat cerebral cortex (Table 2); AF261. The two-site competition curves of this compound with $[^3H]$PZ was converted to single mass-action curves in the presence of the stable GTP analog, GppNHp (not shown). Similar observations on GppNHp sensitivity were observed for competition with $[^3H]$NMS (not shown). This indicates that these compounds behave as efficacious agonists for M1 receptors in rat cerebral cortex. This is in contrast with AF102B competition assays which typically yield mass-action curves. These observations may suggest some differences between recognition of rat cortex M1 receptors by AF102B and these new compounds.

AF267 exhibited single-site competition curves with both $[^3H]$NMS and $[^3H]$PZ in rat cerebral cortex membranes. Studies of phosphoinositides (PI) hydrolysis and arachidonic acid release described below indicated that this compound is a partial agonist. The mass-action binding pattern was therefore similar to data for the M1-selective agonist AF102B to muscarinic receptors in this preparation. We have previously demonstrated that treatment of rat cerebral cortex membranes with 0.1 mM CuSO4 may expose cryptic agonistic binding properties of AF102B (Fisher et al, JPET 257:392-403. 1991; the published data employed competition of AF102B with $[^3H]$QNB, but similar observations were obtained using $[^3H]$NMS and $[^3H]$PZ). Also, such treatment may increase the proportion of high affinity sites for certain agonists, which already exhibit high-affinity sites without $Cu^{2+}$ treatment. We have therefore studied the binding properties of AF267 following $Cu^{2+}$ treatment. Such treatment followed by competition assay with $[^3H]$PZ exposed 18% high affinity sites for AF267 ($K_H$ = 22 nM, i.e. about 100-fold higher affinity compared with $K_L$ = 2.5 µM) without significant change in the low affinity sites. This observation suggests that AF267 may bind to muscarinic receptors in rat cerebral cortex similarly to to AF102B. The ability of $Cu^{2+}$ treatment to expose the high-affinity sites for certain partial agonists may be related to stabilization of receptor/G-protein interaction by coordination complexes of the metal ions with crucial sulfhydryls on these macromolecules(Gurwitz et al, BBRC 1984, 120:271-277). In control membranes this interaction is apparently not favored in the presence of partial agonists, which thus do not exhibit a high-affinity site in competition assays with labelled antagonists.

The ability of compounds to displace $[^3H]$oxotremorine-M { $[^3H]$OXO-M} binding provided a measure of affinity

for the high affinity agonist state of the receptor. The ratio of the Ki values for the displacement of [$^3$H]NMS or [$^3$H]QMB and [$^3$H]OXO-M is used in the literature to predict efficacy. Ratios greater than 100 are associated with full agonists; antagonists give ratios close to unity and intermediate values indicate partial agonists (Orlek et al., J. Med. Chem. 34: 2726, 1991).

Test No. 2. Second messenger activations in brain slices and in cell cultures.

In a procedure for measuring the efficacy of the tested compounds as agonists on the M1 muscarinic receptors, brain slices from rat cortex (200µM cubes) are prepared. For phosphoinositides (PI) turnover assay, these brain slices are loaded with [$^3$H]inositol (4 µCi/ml) by incubating them in Krebs balanced salt solution containing the labeled ligand for 1h at 37°C under oxygenation. After washing, 50µl aliquots are added to each tube containing 10mM LiCl in fresh Krebs solution with or without the tested compound. Following incubation for 20 min at 37°C. the reaction is terminated and labeled products are separated on AG-1-X8 columns as described by Berridge (Biochem. J. 258, 849-858, 1983). Partial agonists, according to the compound tested, produced a less than 80% activation of PI hydrolysis as compared with CCh (a full agonist).

Cell cultures enriched in one subpopulation of muscarinic receptors are used to evaluate second messenger activations by the tested compounds. For PI turnover studies the method of Berridge (Biochem. J. 258:849-858, 1983) is used; for arachidonic acid mobilization studies cells are labeled for 16h with 0.2 µCi/ml of tritiated-arachidonic acid in original growth media. Prior to assay, cells are washed a total of six times with serum-free DME supplemented with HEPES (20mM) and bovine serum albumin (1 mg/ml). Following the washing procedure, 0.5 ml of the same medium are added with ensuing addition of the tested ligands. Assays are terminated by transferring the media to Eppendorf tubes and centrifuging for 10min at 6000 g. Radioactivity in supernatants is counted and presented as dpm tritiated-arachidonic acid released per well. Cyclic AMP accumulation in intact cells is evaluated according to the method of Pinkas-Kramarski et al, Neurosci. Lett. 108:335-340, 1990, whereas adenylyl cyclase activity in isolated membranes is determined according to Johnson and Salomon (Methods in Enzymology Vol 195. R.A. Johnson and J.D. Corbin, eds. Academic Press, pp 3-21, 1991).

Compounds of Formula I with a maximal rate of PI turnover and/or arachidonic acid mobilization (but no significant activation of adenylyl cyclase) higher than 25% are preferred. Some examples for such activity can be found in AF261, AF263, AF266, AF267. These compounds are'capable of activating M1 muscarinic receptors.

Unlike acetylcholine, CCh, oxotremorine-M and other classical full agonists, these compounds induce selective activation of distinct signalling via M1 (or M3) muscarinic receptors. In particular, the selective activation by muscarinic agonists of PI hydrolysis without (or with minimal) activation of cAMP accumulation is the general pattern of activity of the new compounds. These observations may imply induction of the M1 muscarinic receptor-coupling to distinct G-proteins by these selective muscarinic ligands. Thus in addition to the activation of the M1 receptors, these compounds are also selective at the level of distinct secondary messengers. This concept of select activation of only distinct G-proteins via the same muscarinic receptor using selective muscarinic ligands was recently described by the inventors under the concept of ligand-selective signaling employing CHO cells transfected with the rat m1AChR (Fisher et al. Biorganic & Medicinal chem. Lett. 2:839-844, 1992) and in a neuronal type cell line, e.g. PC12M1 cell line (Soc. Neuroci. Abs. Nov. 1993). The possible relevance of this signaling pathway for the development of cholinergic replacement therapy for Alzheimer's disease (AD) is evident in view of findings on elevated Gs levels in AD patients and aged brains (Harrison et al., Mol. Brain Res. 10:71. 1991; Young et al., Dev. Brain Res., 61:243, 1991). Thus compounds from the present invention can be important for the treatment of Alzheimer's disease.

Some of the compounds are full agonists while others are partial agonists when compared with CCh in stimulating PI hydrolysis in cell cultures transfected with m1AChR. Partial agonists include : AF267. AF261, AF263 and AF266 had full agonistic activities in this assay. Concentration response curves for AF261 indicated that maximal activities were obtained at 10 µM and 100 µM, respectively. AF267 behaved as partial agonists, with maximal activities of 75% and 66% relative to 1 mM CCh, respectively. Of the enantiomers of AF267, the more active is AF267B, the (-)-enantiomer; this compound activates PI hydrolysis in cells transfected with the ml receptors even at 10µM (40% of CCh) and at 100µM (80% of CCh).

We tested the potency of the new compounds to induce desensitization of the CCh-mediated PI hydrolysis signal. AF261 induced a smaller desensitization response compared with CCh: following a 24 hour exposure to 100 µM or 1 mM AF261, the PI hydrolysis response to stimulation with 1 mM CCh was decreased by 45% as compared to a reduction by 60% following pre-incubation with 1 mM CCh. AF267, a compound which presented partial agonistic activity in inducing PI hydrolysis. was less capable in inducing desensitization. Thus, following an overnight incubation with 1 mM AF267, the original CCh-mediated PI response was reduced by merely 29%.

We also tested the new compounds for their ability to induce PI hydrolysis in cells transiently transfected with the human m1AChR, m3AChR or m5AChR, in parallel experiments as described by Pittel and Wess (Mol. Pharmacol. 45:61-64, 1994). AF267 exhibited the highest selectivity towards the m1AchR subtype, being about 100-fold more

potent for m1AChR as compared to m3AChR-transfected cells ($ED_{50}$ values were 1.5 and 150 μM, respectively). This is particularly evident when comparing the signal induced by 10μM AF267, which is already maximal in m1AChR-transfected cells, but is only 15% of the maximal CCh signal in m3AChR-transfected cells. AF265 was somewhat more potent and more efficacious in m1AChR than in m3AChR transfected cells and much less active in m5AChR. Similar results were obtained with AF267 and in particular for its more active enantiomer, the (-)-enantiomer, in cell cultures stably transfected with m1 AChR and m3 AChR, respectively.

Arachidonic acid (AA) release is another biochemical pathway that is activated by agonists of the m1AChR. Since this biochemical pathway may be linked to the m1AChR via a different G protein than the PI hydrolysis, we also tested the new compounds in this assay, employing the cell cultures stably transfected with rat m1AChR. AF263, AF266 and AF267 (at 1 mM) showed partial agonistic activities in this assay. Of the enantiomers of A267, the active enantiomer is A267B, the (-)-enantiomer, which is a full agonist pn AA release at 0.1 and 1 mM. AF260 and AF261 showed full agonistic activities compared with 1 mM CCh, in agreement with observations of PI hydrolysis in the same cell line. We observed a tendency of these agonists to induce more AA release compared with 1 mM CCh. This may indicate that the CCh-mediated signal is already undergoing desensitization during the assay period (20 min at 37°C). It is plausible that this desensitization is smaller with the tested compounds. Alternatively, certain tested compounds may be more efficacious than CCh as M1-agonists, i.e. "super-agonists". In that case, it is not clear why this property was not observed in the PI hydrolysis assay employing AF261.

Pre-incubation of cell cultures transfected with m1AChR with 1 mM CCh for 3 h followed by extensive wash-out of the ligand (6 x 1 ml) reduced the AA release considerably. The basal AA release was minimally affected, indicating that no residual CCh was present following the wash-out. For longer incubation periods, we preferred drug concentrations of 100 μM. as concentrations of 1 mM have no physiological relevance for long-term treatments. Following pre-incubation with 100 μM CCh for 24 hours the AA response was almost completely lost. We compared the ability of the new compounds to desensitize the CCh-mediated AA release signal. Stimulation with AF260, AF261 (at 100 μM for 24 h) considerably reduced the CCh-mediated AA release signal. Similar pre-incubations with AF267 resulted in relatively small interference with the subsequent CCh-mediated AA release signal, again pointing to the similarity between these two compounds.

Test 3: Neurotrophic-like effects in cell cultures

Activation of M1 receptors by agonists can lead to synergistic effects with nerve growth factor (NGF) in certain cell cultures enriched with m1 receptors, e.g. PC12 (rat pheochromocytoma cells) transfected with the rat ml AChR (PC12M1 cells), (Pinkas-Kramarski et al, J. Neurochem. 59:2158-2166, 1992).

It has now been discovered, in accordance with another aspect of the invention, that compounds of Formulae I-IX with a maximal rate of PI turnover higher than 25% can synergize the neurite outgrowth produced by NGF. Two classes of compounds can be detected among the compounds of the invention: 1) compounds like AF261 and AF263 which in the absence of NGF induced neurite outgrowth almost similarly to CCh; and 2) those compounds, which in sharp contrast to oxotremorine, do not promote neurite-outgrowth or induce only minimal morphological change in the absence of NGF. Both classes are important for the treatment of AD. In the second class no axonal growth would take place uncontrollably. Therefore, a favorable drug candidate for the treatment of AD, for example, would induce neuritogenesis only under strict control of locally synthesized and released growth factors, such as NGF, brain-derived nerve factor (BDNF), NT-3 etc.

The neurotrophic-like activity of those agonists of the present invention in the neuronal cells, which is also dependent on the presence of NGF, probably indicates that these compounds exerts neurotrophic activities in conjunction with some signal(s) mediated via NGF receptors. One of the possibilities is that these effects are indirect via mlAChRs-mediated increased release of amyloid precursor proteins (APP), vide infra. Notably, endogenous APP are necessary for normal growth of fibroblastic cells, and exogenous APP can stimulate proliferation of these cells (Saitoh et al. Cell, 58:615, 1989; Mattson et al., TINS 16:409-414, 1993). Interestingly, the secreted forms of APP, among other activities, are known to regulate neurite outgrowth and to promote neuronal survival (reviewed by Mattson et al., TINS 16:409-414, 1993). Neuronal cell-death in AD probably involves decreased production and/or availability of neurotrophines, which is turn compromises survival of cholinergic neurons. If the neurotrophic-like events induced by the M1 agonists also occur in the brain this might have a most important clinical significance and thus may indicate a novel treatment for AD.

Test 4: Amyloid precursor protein (APP) secretion in brain slices and cell cultures

There is growing evidence that β-amyloid is a contributor to the pathological process leading to AD. Based on the "Amyloid cascade hypothesis" AD results from the mismetabolism of amyloid precursor protein (APP) (Mattson et al., TINS 16:409-414, 1993). Activation of mAChRs, and in particular the ml subtype, increases secretion of APP in

vitro (Kitsch et al. Science 258:304,1992; Buxbaum et al, PNAS US 89:10075, 1992; Lahiri et al, Biochem. Int. 28:853, 1992). Thus, according to a further aspect of the invention, compounds of Formula I, in particular those showing selective ml agonistic activity and increased release of APP, can be beneficial not only in the treatment of AD, but in delaying its progression or even in preventing AD.

The method employed for evaluation of APP secretion is the Western immunoblotting technique (Nitsch et al., PNAS 90:5191-5193, 1993). We have employed the 22C11 monoclonal anti-APP, which is widely used for detection of APP. This antibody is directed against the amino-terminal region of APP, and identifies all the APP isoforms reported to date. It does not detect amyloid or c-terminal APP fragments formed following APP secretion. For evaluating the effects of the new compounds on APP release, cell lines transfected with ml receptors were cultured in 12-well plates (in some experiments, in 6-well plates) as described by Pinkas-Kramarski et al. (J. Neurochem. 59:2158, 1992). Ligands were added from sterile 100x stock solution for tested periods. Experiments were terminated by washing the plates twice in serum-free medium. Cells were scraped off the plates in cold phosphate/saline buffer (pH = 7.4; PBS) buffer. Following centrifugation (10 min, 10000g) the supernatants were discarded and the pellets were suspended in 0.1 ml cold lysis buffer containing protease inhibitors cocktail (50 mM TRIS:HCl (pH = 7.4), 150 mM NaCl, 5 mM EDTA, 1% Triton X-100, 0.1 mM PMSF, 5 Units/ml aprotinin, 5 μg/ml pepstatin A, 5 μg/ml leupeptin). The pellets were then sonicated for 5 sec at maximal setting (Branson sonicator, model 130) and centrifuged again. The supernatants (cell membrane extracts) were transferred to clean tubes. Samples were taken for protein determination by the Lowry method.

For APP assay equal amounts of protein (typically 100 μg/lane) were diluted in sample buffer containing 0.6% SDS and 1% 2-mercapto-ethanol, and loaded on 10% acrylamide/SDS minigels (Hoeffer Scientific). Pre-stained molecular weight standards (Sigma) were loaded on each gel. Gels were run at a constant current of 25 mAmp/gel at 4°C. Blotting was performed at 100 mAmp for 16-18 h at 4°C, using a nitrocellulose membrane. The following steps were carried out at room temperature. Membranes were blocked for 1 h by immersing in PBS containing 10% low-fat milk. This step was followed by washing 3x5 min in PBS containing 0.05% Tween-20 and 0.1% BSA (PBST). Anti-APP monoclonal antibody, clone# 22C11 frod Boehringer Manneheim (cat. # 1285-262) was added at a dilution of 1:200 (0.25 μg/ml IgG)in PBST buffer for 2 h at RT or 18 h at 4 °C. In some experiments, similar blots were probed with control mouse IgG at a similar concentration. This step was followed by 3x5 min washing steps in PBST. The secondary horseradish peroxidase-linked goat antimouse IgG antibodies (from Jackson Immunochemicals; cat. #115-035-003) were used at 1:2000 in PBST for 1 h. followed by washing as above. Staining of immunoblots was performed at room temperature for 15 - 30 min using a fresh solution of 4-chloronaphthol (0.2 mg/ml in TBS containing 16% ethanol) and $H_2O_2$ (0.01%). Stained blots were photographed (Kodak TMAX negative film) and scanned in an LKB UltroScan KL laser scanner set at 40 μm vertical interval size and 2.4 mm horizontal slit width. Each lane was scanned three times at slightly different positions; data obtained was the mean optical density values.

By this method we measured the amount of APP remaining in the cell membrane fraction following incubation (typically for 24 h) with the tested compounds. The smaller APP levels remaining in the membranes following such incubation with agonists were taken as an indication that more APP was secreted by the cells. This type of measurement is relevant when considering the ability of muscarinic agonists to decrease accumulation of β-amyloid in Alzheimer disease, because only membrane associated APP. unlike secreted forms of APP. may give rise to β-amyloid. For measuring the APP which is secreted to the cell culture medium ("conditioned medium") we had to concentrate the conditioned medium, using Amicon microfiltration membranes (30 kDa cutoff). Following the concentration step. protein amounts in the samples were measured by the Bradford method (Bio-Rad kit # 500-0006) using bovine gamma-globulin as a standard. Equal protein amounts were loaded on poly-acrylamide gel electrophoresis (PAGE). The amount of APP in the samples was analyzed by immunoblotting with the 22C11 monoclonal anti-APP antibody, as described above. Molecular weights were estimated from pre-stained standards (Sigma) which were included in each gel.

Compounds of Formula I with a maximal rate of APP secretion in cell cultures higher than 125% (over basal taken as 100%) are preferred. Such examples for such activity can be found in AF261, AF263, AF267.

To enable the processing of a larger number of samples simultaneously, some studies were performed using a dot-blot technique (96 dots may be processed simultaneously on a single membrane). In this technique, conditioned medium from stimulated PC12M1 cells is applied directly to nitrocellulose membranes under vacuum (without prior concentration). The membranes are then probed similarly to the immunoblotting protocol. Detailed concentration-response and time-dependency studies were also analyzed by separation of the secreted APP on PAGE. The results of these studies and those obtained from the dot-blot studies are in good agreement. Studies of APP secretion by cells transfected with m1AChR were thus performed with the new compounds and employing also the dot-blot technique and 1 h incubation. Some of the compounds such as AF261, AF263, and AF267 are full agonists in this assay.

Desensitization of m1AChR-mediated APP secretion is stronger during prolonged incubations with CCh compared with partial agonists from this invention. This explanation is especially attractive. when considering our data on desensitization of the arachidonic acid (AA) release signals in combination with the recent suggestion of Emmerling et al. (BBRC 197: 292-297, 1993) that AA release is involved in stimulation of APP secretion. Our data would indicate

that some of the new compounds may be favored over the highly efficacious agonists for clinical use to lower cell-associated APP levels over long periods. Compound which exhibited minimal desensitization in the AA release assay is AF267. Measurements of cell-associated APP following incubations with AF267 for 24 h (both at 100 μM) indicated decreased APP levels, which were stronger with AF267 and almost similar to the effect of CCh.

The data on APP secretion by cell cultures stably transfected with m1AChR clearly demonstrate that several of the new compounds may stimulate APP secretion, and may therefore contribute to decreased amyloid accumulation in vivo. However, to allow an extrapolation of the tissue-culture data to an in vivo situation, it should preferably be demonstrated that the compounds may also stimulate APP secretion in the brain. To that end, APP secretion by rat cerebral cortex slices was studied. In a typical experiment, rat cerebral cortex slices were prepared immediately from freshly-dissected tissue, minced in two perpendicular directions by a McIlwain Tissue Chopper (300×300 micron) and rinsed three times with oxygen-saturated Krebs buffer. Washed slices were equilibrated with this buffer for 50 min at 37°C. Following the equilibration step (which is required for removal of cell debris resulting from the slicing protocol), the buffer was changed to Krebs buffer containing 50 μg/ml BSA (as a protein carrier) and proteases inhibitors cocktail (0.1 mM PMSF and 5 μg/ml each leupeptin, pepstatin and aprotinin-A). The slices were dispensed to plastic tubes and stimulated for 1 h at 37 °C (2 h in one experiment) with the indicated concentrations of the tested compounds. At the end of the incubation period. the buffer samples were collected by centrifugation, analyzed for protein content, and equal protein amounts were separated and processed for immunoblotting with 22C11 anti-APP monoclonal antibody, using PAGE similarly to the experiments with cultured cells. Secreted APP appeared as two protein bands, having apparent molecular weights of 117 kDa (major band) and 90 kDa (minor band). These APP bands may correspond to secreted forms of APP751 and APP695 (the major. Kunitz-containing and Kunitzdeficient forms of APP). Alternatively, they may correspond to mature and immature (non-glycosylated) forms of secreted APP751). At present, sufficient data to distinguish between these possibilities is unavailable, because the 22C11 monoclonal antibody employed in these studies identifies all forms of secreted APP. Both CCh or AF267, for example (both at 0.1 mM) increased APP secretion by rat cerebral cortex slices by 2 - 3-fold compared with control APP secretion. This was evident for both the 117 kDa and the 90 kDa protein bands.

The observations on APP secretion by cerebral cortex slices are unique. To date, the only published demonstration of APP secretion by brain tissue in-vitro is by Nitsch et al. (PNAS 90:5191-5193, 1993) who reported that electrical stimulation of rat hippocampal slices increased APP release. Notably, their study did not include stimulation with a receptor agonist. Our observations therefore are innovative and novel, as they are the first demonstration of regulation of APP secretion in brain tissue by any receptor ligand, and in particular, an M1 agonist like AF267. Those compounds which stimulate APP secretion in rat cortical slices are preferred. Such compounds can be considered all those agonists in this invention capable of stimulating second messengers like PI >25% control. These unique observations may indicate the potential of some of the compounds for slowing the deposition of A4-amyloid peptides in brains of Alzheimer's disease patients.

Test 5: Tau protein phosphorylation in cell cultures transfected with the m1AChRs and in rat cortical slices

Tau (τ) is a neuron-specific microtubule-associated protein which is abundant in axons. Tau is expressed as several isoforms, all of which are derived by alternative splicing of a single gene (human tau has 6 isoforms, having 352 - 441 amino acids). It functions in stabilization of microtubules of neuronal axons; its binding to microtubules is regulated by its phosphorylation in distinct sites (Mandelkow and Mandelkow, TIBS 18:480-483, 1993). This binding, in turn, regulates axonal growth and stability (Baas et al., J. Cell Biol. 115:1333-1344, 1991; Mattson, Brain Res. 582: 107-118, 1992). One of the hallmarks of Alzheimer's disease, in addition to amyloid deposition in plaques, is accumulation of tau or tau-derivatives in neurofibrillary tangles (NFTs). These tangles probably reflect the end-product of neuronal cell death in the diseased brain tissue. Numerous studies have demonstrated changes in tan protein phosphorylation in post-mortem brain samples of AD patients. This phenomena is best documented as increased immunoreactivity with the monoclonal antibody ALZ-50, which was shown to detect hyper-phosphorylated forms of tau in these brain samples (Vincent and Davies, PNAS 87:4840-4844, 1990; Vincent and Davies, Brain Res. 531:127-135, 1990). It was therefore suggested that alterations in the delicate balance between tau-specific kinases/phosphatases may be involved in the process of neuronal cell death in AD, and therefore a correction of such probable imbalance may be of potential therapeutic value.

Accordingly, the phosphorylation level of tau proteins in cell cultures stably transfected with m1AChR following stimulation with CCh or one of the tested compounds, was studied. This was done by employing the monoclonal antibody tau-1, which was shown to recognize only de-phosphorylated, but not phosphorylated, isoforms of tau (Mandelkow and Mandelkow, TIBS 18:480-483, 1993). Following incubations of the cells or rat cortical slices with the agonists for various periods, the cells or slices were washed three times with PBS, scrapped in PBS containing 0.2 mM EDTA, and the centrifuged 5 min at 10,000xg. The membrane pellets were analyzed for protein content, and equal protein amounts were loaded on PAGE for immuno-blotting with the tau-1 antibody. Measurements of immuno-reactivity

were carried out similarly to the studies of APP (employing video-densitometry), with the exception that the peroxidase-coupled second antibody was detected using the Enhanced Chemi-Luminescence (ECL) technique (kit RPN-2109; Amersham, UK).

Notably, both CCh or the tested agonists increased tau1 immuno-reactivity, and this increase was completely blocked by atropine. A striking increase in CCh-mediated tau-1 immuno-reactivity was observed in cells cultured with NGF (50 ng/ml) for 3 days prior to stimulation with the muscarinic ligands. Similar experiments employing varying concentrations of CCh or the tested agonist indicated that the increased tau-1 immuno-reactivity required relatively high ligand concentrations (10 - 100 μM).

Tau phosphorylation in rat cerebral cortex slices following stimulation with CCh in comparison with the present compounds, was also measured. Surprisingly, again the muscarinic agonists tested are capable of decreasing tau phosphorylation.

Thus, muscarinic agonists are capable of decreasing tau phosphorylation by activating the transfected m1AChR in cell cultures and more importantly in the brain slices. Also, the effects of these agonists seem to be long-lasting, being evident even following incubations of the cells with 100 μM ligands for at least three days. These observations may indicate that selective M1-agonists, are capable of decreasing tau phosphorylation.

These observations are novel, as to the best of our knowledge, no relationship was demonstrated to date between the cholinergic . deficiency in Alzheimer's disease and the accumulation of phosphorylated tau protein isoforms in the neurofibrillary tangles typical to the diseased brain. These new observations may have relevance to the slowing of the progression of Alzheimer's disease by M1 agonists.

In conclusion:

M1-Agonists can have a number of effects. Thus, surprisingly, the mechanisms of action of m1 agonists is more complex than originally envisaged. The following activities have already been associated with m1 agonists, in vitro (and perhaps in vivo):

1. Binding with and activation of m1 receptors;
2. Distinct activation of select G-proteins (e.g., Gq but not Gs);
3. Neurotrophic-like and synergistic effects with NGF;
4. Secretion of the amyloid precursor protein (APP) and decrease of β-amyloids;
5. Increasing the proportion of dephosphorylated τ proteins;
6. NGF-like effects.

All these effects can be interconnected in a model in which activation of ml AChRs leads to a cascade of related events. These complex mechanisms underlying ml agonistic activity can be linked into a general scenario which explains the abnormal APP processing, NGF-like deficits and cholinergic deficits in SDAT and AD. Consequently, ml agonists like those of the present invention may be of value in preventing amyloid formation by promoting selectively and positively the secretase processing pathway, and may also promote the action of neurotrophines in AD, due to their synergistic effect with NGF. Thus, ml agonists may be useful in a cholinergic replacement strategy and also in delaying the progression of AD. The present compounds which are ml agonists are (unlike peptides) relatively small molecules, which have neurotrophic-like effects and promote the release and normal processing of APP, and increase the dephosphorylated (or decrease the phosphorylated) tau proteins. Thus, treatment of cells with an ml agonist might possibly shift the processing of APP from amyloidogenic lysosomal pathway to normal secretory pathway and consequently cholinergic therapies of AD may have longer term effects on β-amyloid deposition (re also Lahiri et al. Biochem. Int. 28: 853-860, 1992). Furthermore, since some of the agonists from this invention show NGF-like effects in a controlled manner and in particular in the presence of NGF, such compounds can be of significant value in the future treatment of AD/SDAT. In this way neurite outgrowth can be better controlled. Moreover, our compounds have a long-lasting beneficial effect on neurite outgrowth, in presence of NGF. Thus it can be speculated that the future possible treatment using such growth factors like NGF for AD/SDAT patients, might require less frequent administrations of the growth factor itself, when administered together with an ml agonist. Notably, the administration of NGF to the brain is a most difficult task since this compound does not cross the blood-brain barrier. Once this problem will be solved (e.g. via special delivery systems) it will still require repeated administration of NGF-like compounds. ml Agonists in this regard can reduce the number of the required repeated administration of NGF, since such agonists prolong the effects of NGF (at least, in vitro). Interestingly, clinical trials of NGF (icv) in 2 AD patients showed very serious adverse effects (serious pain, confusion, deterioration of mini-mental score), (3rd Springfield Symposium on Alzheimer's Disease, May 11-15, 1994, Springfield, Il., USA). The use of M1 agonists like those in the present Invention which synergize with NGF can be of great value in reducing the side-effects associated with NGF in humans, since then lower doses of NGF could be used.

Test No. 6: Pharmacological and toxicological profiles

The study was carried out by observing the animals after iv or oral (mice) or oral (intra gastric for rats) administration of 3-6 dose levels of each substance. Results are summarized in Table 3.

At different time intervals post-administration (10, 20, 30, 45, 60, 120, 240 min and 24 hr) animals were subjected to detailed observations of changes in general behavior, reflexes and autonomic effects. Mortality was recorded at 24 hr post administration of the test compounds. Body temperature in rats was measured using Tele-Thermometer (Model 46 TUC). The various pharmacological and behavioral parameters included: salivation, redness around the nose and mouth, chromodacryorrhea, sedation, ataxia, cyanosis, tremors, convulsions, hypothermia, opisthotonos, respiratory distress, diarrhea, gnawing, piloerection, mortality, changes in pupil diameter, rotarod, hypo- or hyper-activity and vocalization. Some of the tested compounds are non-toxic up to 500 mg/kg (p.o., mice & rats).

Table 3:

| Pharmacolorical and toxological profiles in mice or rats | | | | | | | | | | | | | | | |
|---|---|---|---|---|---|---|---|---|---|---|---|---|---|---|---|
| Cpd. AF- | Dose*: | 1 | 2 | 3 | 4 | 5 | 6 | 7 | 8 | 9 | 10 | 11 | 12 | 13 | 14 |
| 261 | 3.75 | 0/4 | 0/4 | | 0/4 | 0/4 | 0/4 | 0/4 | 0/4 | | 0/4 | 0/4 | 0/4 | 0/4 | 0/4 |
| | 7.52 | 0/4 | 0/4 | | 0/4 | 0/4 | 0/4 | 0/4 | 4/4 | | 0/4 | 0/4 | 0/4 | 0/4 | 0/4 |
| rats | 15.6 | 3/4 | 4/4 | | 4/4 | 0/4 | 0/4 | 0/4 | 4/4 | | 0/4 | 3/4 | 0/4 | 0/4 | 0/4 |
| | 62.5 | 4/4 | 4/4 | | 4/4 | 0/4 | 4/4 | 0/4 | 4/4 | | 0/4 | 3/4 | 3/4 | 3/4 | 0/4 |
| | 250 | 4/4 | 4/4 | | 4/4 | 0/4 | 4/4 | 4/4 | 4/4 | | 0/4 | 1/4 | 4/4 | 4/4 | 4/4 |

*mk/kg, administered orally unless stated otherwise

KEY TO TABLE 3

1 = salivation

2 = chromodacryorrhea (rats)
or lacrimation (mice) 3 = sedation

4 = ataxia

5 = cyanosis

6 = tremors

7 = convulsions

8 = hypothermia

9 = mydriasis

10 = respiratory distress

11 = diarrhea

12 = gnawing

13 = piloerection

14 = mortality

Discussion of results

AF261

In rats, hypothermia (a typical CNS effect) was the only symptom produced by 7.5 mg/kg of AF261. It became evident within 10 min after administration and lasted for 50 min. After increasing the dose to 15.6 mg/kg, additional symptoms such as salivation, chromacyorrhea, hypoactivity, ataxia and diarrhea were apparent within 10 min. At higher doses and up to 250mg/kg, additional signs such as redness around the nose and mouth, tremors, convulsions, opisthotonos, gnawing and piloerection were apparent. These signs could be detected 5-10 min post administration. The severity and the extent of most of those symptoms were dose-related. Mortality of 4/4 rats occurred only at the highest dose tested (250mg/kg), 45 min after administration.

The following correlation is provided for examples of specific compounds according to the invention, according to IUPAC systematic nomenclature, with their code numbers as used herein: 2-ethyl-4,8-dimethyl-1-thia-4,8-diaza-spiro[4.5]decane-3-one (AF272); 2-methyl-1-thia-4,8-diaza-spire[4.5]decan-3-one (AF263); 2,4,8-trimethyl-1-thia-4,8-diaza-spiro[4.5]decan-3-one (AF266);

While the present invention has been particularly described herein, with especial reference to exemplified and otherwise specified embodiments, persons skilled in the art will be aware that many variations and modifications may be made. The invention is accordingly not to be construed as restricted to such embodiments which have been particularly described.

## Claims

1. The use of a compound of the formula (I) in the preparation of a medicament for use in the treatment of a disease of the central or peripheral nervous system in a mammal, the formula (I) being:

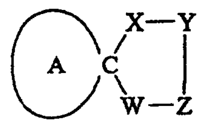

wherein X is >S, Y is >CAR', Z is >C=O and W is >NR°,
in which R is selected from H, $C_{1-6}$-alkyl, $C_{1-6}$-alkoxy, $C_{2-6}$-hydroxyalkyl, $C_{2-6}$-alkenyl, $C_{2-6}$-alkynyl, phenyl and $C_{1-6}$-alkyl substituted by 1, 2 or 3 phenyls;
R' is selected from H, $C_{1-6}$-alkyl, $C_{1-6}$-alkoxy, $C_{2-6}$-hydroxyalkyl, $C_{2-6}$-alkenyl, $C_{2-6}$-alkynyl, phenyl and $C_{1-6}$-alkyl substituted by 1, 2 or 3 phenyls; and
$R^0$ is selected from H, $C_{1-6}$-alkyl, $C_{1-6}$-alkoxy, $C_{2-6}$-hydroxyalkyl, $C_{2-6}$-alkenyl, $C_{2-6}$-alkynyl, phenyl, $C_{1-6}$-alkyl substituted by 1, 2 or 3 phenyls, and $C_{1-6}$-alkanoyl; and
A is a ring (M):

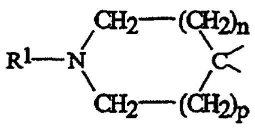

which is unsubstituted or is substituted by 1 - 3 substituents selected from $C_{1-6}$-alkyl and hydroxyl, and wherein n and p are each 0, 1, 2 or 3, provided that n + p is 1 - 3; and $R^1$ is selected from H, $C_{1-6}$-alkyl, $C_{2-6}$-alkenyl, $C_{2-6}$-alkynyl, $C_{3-7}$-cycloalkyl, $C_{1-6}$-alkyl substituted by 1 - 6 halogen atoms, hydroxy-$C_{1-6}$-alkyl, $C_{1-6}$-alkoxy, $C_{1-6}$-alkylthio, $C_{1-6}$-alkoxy-$C_{1-6}$-alkyl, carboxy-$C_{1-6}$-alkyl, ($C_{1-6}$-alkoxy)carbonyl-$C_{1-6}$-alkyl, amino-$C_{1-6}$-alkyl, mono-($C_{1-6}$-alkyl)amino-$C_{1-6}$-alkyl, di-($C_{1-6}$-alkyl)amino-$C_{1-6}$-alkyl, 2-oxo-pyrrolidin-1-yl-methyl, aryl, diarylmethylol, $C_{1-6}$-alkyl substituted by one or two aryl groups, $C_{1-6}$-alkanoyl and arylcarbonyl, where aryl denotes unsubstituted phenyl or phenyl substituted by 1 - 3 substituents selected from halogen, $C_{1-6}$-alkyl, $C_{1-6}$-alkoxy and $CF_3$; including pharmaceutically acceptable salts, quaternary compounds structurally derived from the compounds at a tertiary nitrogen atom, enantiomers and racemates thereof.

2. The use of a compound according to claim 1, for use in the manufacture of a medicament having biological activity selected from (a) muscarinic agonist activity, (b) neurotrophic-like or synergistic activity with NGF, (c) amyloid precursor protein (APP) secreting activity and β-amyloids decreasing activity, (d) activity increasing the proportion of dephosphorylated τ proteins and (e) NGF-like activity.

3. The use according to claim 1 wherein the compound of formula (I) is of the formula (K):

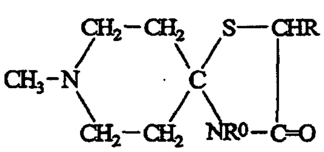

wherein R is selected from H, $C_{1-6}$ alkyl $C_{1-6}$ alkoxy, $C_{2-6}$ hydroxyalkyl, $C_{2-6}$-alkenyl, $C_{2-6}$ alkynyl, phenyl and $C_{1-6}$ alkyl substituted by 1, 2 or 3 phenyls, and
$R^0$ is selected from H, $C_{1-6}$ alkyl $C_{1-6}$ alkoxy, $C_{2-6}$ hydroxyalkyl, $C_{2-6}$-alkenyl, $C_{2-6}$ alkynyl, phenyl, $C_{1-6}$ alkyl substituted by 1, 2 or 3 phenyls, and $C_{1-6}$ alkanoyl;
including pharmaceutically acceptable salts, quaternary compounds structurally derived from the compounds at a tertiary nitrogen atom, enantiomers and racemates thereof.

4. The use according to claim 3 wherein the compound of formula (K) is AF267 where R is ethyl and R° is hydrogen.

**5.** The use according to claim 4, wherein the compound of formula (K) is the hydrochloride salt of AF267.

**6.** The use according to claim 3 wherein the compound of formula (K) is AF266 where R is methyl and R° is methyl.

**7.** The use according to claim 3 wherein the compound of formula (K) is AF272 where R is ethyl and R° is methyl.

**8.** The use according to any preceding claim wherein the compound of formula (I) is in the form of an enantiomer.

**9.** The use according to claim 7 when dependent on claim 4, wherein the compound of formula (I) is AF267B, that is (-)AF267.

**10.** An enantiomer of a compound of the formula (I):

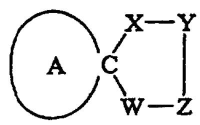

wherein X is >S, Y is >CRR', Z is >C=O and W is >NR,
in which R is selected from H, $C_{1-6}$-alkyl, $C_{1-6}$-alkoxy, $C_{2-6}$-hydroxyalkyl, $C_{2-6}$-alkenyl, $C_{2-6}$-alkynyl, phenyl and $C_{1-6}$-alkyl substituted by 1, 2 or 3 phenyls;
R' is selected from H, $C_{1-6}$-alkyl, $C_{1-6}$-alkoxy, $C_{2-6}$-hydroxyalkyl, $C_{2-6}$-alkenyl, $C_{2-6}$-alkynyl, phenyl and $C_{1-6}$-alkyl substituted by 1, 2 or 3 phenyls; and
$R^0$ is selected from H, $C_{1-6}$-alkyl, $C_{1-6}$-alkoxy, $C_{2-6}$-hydroxyalkyl, $C_{2-6}$-alkenyl, $C_{2-6}$-alkynyl, phenyl, $C_{1-6}$-alkyl substituted by 1, 2 or 3 phenyls, and $C_{1-6}$-alkanoyl; and
A is a ring (M):

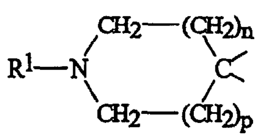

wherein n and p are each 0, 1, 2 or 3, provided that n+p is 1 - 3; and
$R^1$ is selected from H, $C_{1-6}$-alkyl, $C_{2-6}$-alkenyl, $C_{2-6}$-alkynyl, $C_{3-7}$-cycloalkyl, $C_{1-6}$-alkyl substituted by 1 - 6 halogen atoms, hydroxy-$C_{1-6}$-alkyl, $C_{1-6}$-alkoxy, $C_{1-6}$-alkylthio, $C_{1-6}$-alkoxy-$C_{1-6}$-alkyl, carboxy-$C_{1-6}$-alkyl, ($C_{1-6}$-alkoxy)carbonyl-$C_{1-6}$-alkyl, amino-$C_{1-6}$-alkyl, mono-($C_{1-6}$-alkyl)amino-$C_{1-6}$-alkyl, di-($C_{1-6}$-alkyl)amino-$C_{1-6}$-alkyl, 2-oxo-pyrrolidin-1-yl-methyl, aryl, diarylmethylol, $C_{1-6}$-alkyl substituted by one or two aryl groups, $C_{1-6}$-alkanoyl and arylcarbonyl, where aryl denotes unsubstituted phenyl or phenyl substituted by 1 - 3 substituents selected from halogen, $C_{1-6}$-alkyl, $C_{1-6}$-alkoxy and $CF_3$;
including pharmaceutically acceptable salts and quaternary compounds structurally derived from the compounds at a tertiary nitrogen atom.

**11.** An enantiomer according to claim 10, for use in a method of treatment of the human or animal body by therapy.

**12.** An enantiomer as defined in claim 10, for use in the treatment of a disease of the central or peripheral nervous system in a mammal.

**13.** An enantiomer according to claim 12, which is AF267B, that is (-)AF267, being a compound of formula (K):

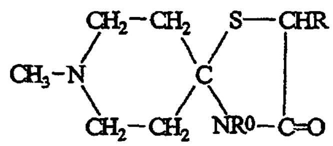

where R is ethyl and R° is hydrogen.

**14.** A compound AF267 which is a compound of formula (K):

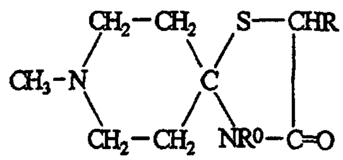

where R is ethyl and R° is hydrogen.

**15.** A compound according to claim 14, which is the hydrochloride salt of AF267.

**16.** A compound AF266 which is a compound of formula (K):

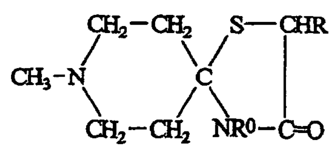

where R is methyl and R° is methyl.

**17.** A compound AF272 which is a compound of formula (K):

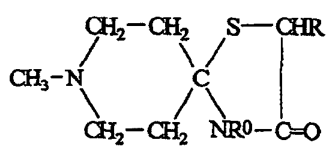

where R is ethyl and R° is methyl.

**18.** A compound AF261 which is a compound of formula (K):

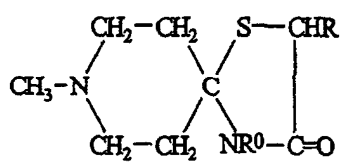

where R is methyl and $R^0$ is hydrogen.

**19.** A pharmaceutical composition comprising an enantiomer according to any of claims 10 to 13, or a compound according to any of claims 14 to 18, together with a pharmaceutically acceptable diluent, carrier or adjuvant.

**20.** A pharmaceutical composition according to claim 19, together with at least one further pharmacologically active compound selected from the group consisting of physostigmine, tetrahydroaminoacridine, choline, lecithin, piracetam, aniracetam, pramiracetam, oxiracetam, 4-aminopyridine, 3,4-diaminopyridine, somatostatin, pirenzepine, N-methylatropine, N-butylscopolamine, scopolamine, clonidine, quanfamicine, propantheline, methantheline, glycopyrrolate, tropenzilium, nortriptyline, amitriptyline, imipramine, minaprine, secoverine, AFDX-116, nicotine, alaproclate, zimelidine, deprenyl and Nerve Growth Factor.

**21.** A pharmaceutical composition according to claim 19 or 20, which is in a form suitable for oral, rectal, parenteral or transdermal administration, or for administration by insufflation or nasal spray, and in the case of transdermal administration the composition may comprise as an additional component, a low molecular weight fatty acid.

**22.** A pharmaceutical composition according to claim 19 for use in treating diseases of the central and peripheral nervous system in mammals, which composition comprises an amount effective for use in treating said diseases of at least one compound as defined in any of claims 1 to 16, together with at least one pharmaceutically acceptable diluent, carrier or adjuvant, and as a further pharmacologically active compound, Nerve Growth Factor, provided that the said at least one compound is present in an amount which promotes the nerve growth activity of the Nerve Growth Factor.

**Patentansprüche**

**1.** Verwendung einer Verbindung der Formel (I) bei der Herstellung eines Medikaments zur Verwendung bei der Behandlung einer Krankheit des zentralen oder peripheren Nervensystems bei einem Säugetier, wobei die Formel (I) ist:

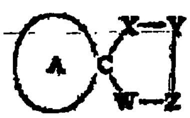

wobei X>S ist, Y>CRR' ist, Z>C=O ist und W>NR$^0$ ist,

in welcher R aus H, $C_{1-6}$-Alkyl, $C_{1-6}$-Alkoxy, $C_{2-6}$-Hydroxyalkyl, $C_{2-6}$-Alkenyl, $C_{2-6}$-Alkinyl, Phenyl und $C_{1-6}$-Alkyl, substituiert mit 1, 2 oder 3 Phenylen, ausgewählt ist;
R' aus H, $C_{1-6}$-Alkyl, $C_{1-6}$-Alkoxy, $C_{2-6}$-Hydroxyalkyl, $C_{2-6}$-Alkenyl, $C_{2-6}$-Alkinyl, Phenyl und $C_{1-6}$-Alkyl, substituiert mit 1, 2 oder 3 Phenylen, ausgewählt ist; und
R° aus H, $C_{1-6}$-Alkyl, $C_{1-6}$-Alkoxy, $C_{2-6}$-Hydroxyalkyl, $C_{2-6}$-Alkenyl, $C_{2-6}$-Alkinyl, Phenyl, $C_{1-6}$-Alkyl, substituiert mit 1, 2 oder 3 Phenylen, und $C_{1-6}$-Alkanoyl ausgewählt ist; und
A ein Ring (M) ist

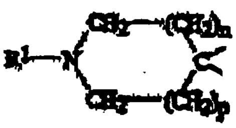

welcher unsubstituiert ist oder mit 1-3 Substituenten, ausgewählt aus $C_{1-6}$-Alkyl und Hydroxyl, substituiert ist und wobei n und p jeweils 0, 1, 2 oder 3 sind, mit der Maßgabe, daß n+p 1-3 ist; und R$^1$ ausgewählt ist aus H, $C_{1-6}$-Alkyl, $C_{2-6}$-Alkenyl, $C_{2-6}$-Alkinyl, $C_{3-7}$-Cycloalkyl, $C_{1-6}$-Alkyl, substituiert mit 1-6 Halogenatomen, Hydroxy-$C_{1-6}$-alkyl, $C_{1-6}$-Alkoxy, $C_{1-6}$-Alkylthio, $C_{1-6}$-Alkoxy-$C_{1-6}$-alkyl, Carboxy-$C_{1-6}$-alkyl, ($C_{1-6}$-Alkoxy)carbonyl-$C_{1-6}$-alkyl, Amino-$C_{1-6}$-alkyl, Mono-($C_{1-6}$-alkyl)amino-$C_{1-6}$-alkyl, Di-($C_{1-6}$-alkyl)amino-$C_{1-6}$-alkyl, 2-Oxopyrrolidin-1-yl-methyl, Aryl, Diarylmethylol, $C_{1-6}$-Alkyl, substituiert mit einer oder zwei Arylgruppen, $C_{1-6}$-Alkanoyl und Arylcarbonyl, wo Aryl unsubstituiertes Phenyl oder Phenyl, substituiert mit 1-3 Substituenten, ausgewählt aus Halogen, $C_{1-6}$-Alkyl, $C_{1-6}$-Alkoxy und $CF_3$, bezeichnet;

einschließlich pharmazeutisch verträglicher Salze, quaternärer Verbindungen, strukturell abgeleitet von den Verbindungen an einem tertiären Stickstoffatom, Enantiomere und Racemate davon.

**2.** Verwendung einer Verbindung nach Anspruch 1 zur Verwendung bei der Herstellung eines Medikaments mit biologischer Wirksamkeit, ausgewählt aus (a) musoarinagonistischer Aktivität, (b) neurotrophartiger oder synergistischer Aktivität mit NGF, (c) Amyloidvorläuferprotein (APP) absondernder Aktivität und β-Amyloide vermindernder Aktivität, (d) Aktivität, die den Anteil von dephosphorylierten τ-Proteinen vergrößert, und (e) NGF-artiger Aktivität.

**3.** Verwendung nach Anspruch 1, wobei die Verbindung der Formel (I) die Formel (K) hat:

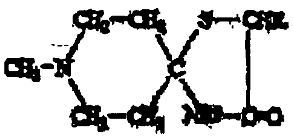

wobei R aus H, $C_{1-6}$-Alkyl, $C_{1-6}$-Alkoxy, $C_{2-6}$-Hydroxyalkyl, $C_{2-6}$-Alkenyl, $C_{2-6}$-Alkinyl, Phenyl und $C_{1-6}$-Alkyl, substituiert mit 1, 2 oder 3 Phenylen, ausgewählt ist, und

$R^0$ aus H, $C_{1-6}$-Alkyl, $C_{1-6}$-Alkoxy, $C_{2-6}$-Hydroxyalkyl, $C_{2-6}$-Alkenyl, $C_{2-6}$-Alkinyl, Phenyl, $C_{1-6}$-Alkyl, substituiert mit 1, 2 oder 3 Phenylen, und $C_{1-6}$-Alkanoyl ausgewählt ist;

einschließlich pharmazeutisch verträglicher Salze, quaternärer Verbindungen, strukturell abgeleitet von den Verbindungen an einem tertiären Stickstoffatom, Enantiomere und Racemate davon.

**4.** Verwendung nach Anspruch 3, wobei die Verbindung der Formel (K) AF267 ist, wo R Ethyl ist und R° Wasserstoff ist.

**5.** Verwendung nach Anspruch 4, wobei die Verbindung der Formel (K) das Hydrochloridsalz von AF267 ist.

**6.** Verwendung nach Anspruch 3, wobei die Verbindung der Formel (K) AF266 ist, wo R Methyl ist und R° Methyl ist.

**7.** Verwendung nach Anspruch 3, wobei die Verbindung der Formel (K) AF272 ist, wo R Ethyl ist und R° Methyl ist

**8.** Verwendung nach einem der vorhergehenden Ansprüche, wobei die Verbindung der Formel (I) in der Form eines Enantiomers vorliegt.

**9.** Verwendung nach Anspruch 7, wenn abhängig von Anspruch 4, wobei die Verbindung der Formel (I) AF267B, das heißt (-)AF267, ist.

**10.** Enantiomer einer Verbindung der Formel (I)

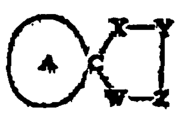

wobei X >S ist, Y >CRR' ist, Z >C=O ist und W >$NR^0$ ist,

in welcher R aus H, $C_{1-6}$-Alkyl, $C_{1-6}$-Alkoxy, $C_{2-6}$-Hydroxyalkyl, $C_{2-6}$-Alkenyl, $C_{2-6}$-Alkinyl, Phenyl und $C_{1-6}$-Alkyl, substituiert mit 1, 2 oder 3 Phenylen, ausgewählt ist;
R' aus H, $C_{1-6}$-Alkyl, $C_{1-6}$-Alkoxy, $C_{2-6}$-Hydroxyalkyl, $C_{2-6}$-Alkenyl, $C_{2-6}$-Alkinyl, Phenyl und $C_{1-6}$-Alkyl, substituiert mit 1, 2 oder 3 Phenylen, ausgewählt ist; und
$R^0$ aus H, $C_{1-6}$-Alkyl, $C_{1-6}$-Alkoxy, $C_{2-6}$-Hydroxyalkyl, $C_{2-6}$-Alkenyl, $C_{2-6}$-Alkinyl, Phenyl, $C_{1-6}$-Alkyl, substituiert mit 1, 2 oder 3 Phenylen, und $C_{1-6}$-Alkanoyl ausgewählt ist; und
A ein Ring (M) ist;

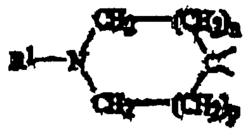

wobei n und p jeweils 0, 1, 2 oder 3 sind, mit der Maßgabe, daß n+p 1-3 ist; und
$R^1$ ausgewählt ist aus H, $C_{1-6}$-Alkyl, $C_{2-6}$-Alkenyl, $C_{2-6}$-Alkinyl, $C_{3-7}$-Cycloalkyl, $C_{1-6}$-Alkyl, substituiert mit 1-6 Halogenatomen, Hydroxy-$C_{1-6}$-alkyl, $C_{1-6}$-Alkoxy, $C_{1-6}$-Alkylthio, $C_{1-6}$-Alkoxy-$C_{1-6}$-alkyl, Carboxy-$C_{1-6}$-alkyl, ($C_{1-6}$-Alkoxy)carbonyl-$C_{1-6}$-alkyl, Amino-$C_{1-6}$-alkyl, Mono-($C_{1-6}$-alkyl)amino-$C_{1-6}$-alkyl, Di-($C_{1-6}$-alkyl)amino-$C_{1-6}$-alkyl, 2-Oxo-pyrrolidin-1-yl-methyl, Aryl, Diarylmethylol, $C_{1-6}$-Alkyl, substituiert mit einer oder zwei Arylgruppen, $C_{1-6}$-Alkanoyl und Arylcarbonyl, wo Aryl unsubstituiertes Phenyl oder Phenyl, substituiert mit 1-3 Substituenten, ausgewählt aus Halogen, $C_{1-6}$-Alkyl, $C_{1-6}$-Alkoxy und $CF_3$, bezeichnet;
einschließlich pharmazeutisch verträglicher Salze und quaternärer Verbindungen, strukturell abgeleitet von den Verbindungen an einem tertiären Stickstoffatom.

**11.** Enantiomer nach Anspruch 10 zur Verwendung in einem Verfahren der Behandlung des menschlichen oder tierischen Körpers durch Therapie.

**12.** Enantiomer, wie in Anspruch 10 definiert, zur Verwendung bei der Behandlung einer Krankheit des zentralen oder peripheren Nervensystems bei einem Säugetier.

**13.** Enantiomer nach Anspruch 12, welches AF267B, das heißt (-)AF267, ist, das eine Verbindung der Formel (K) ist:

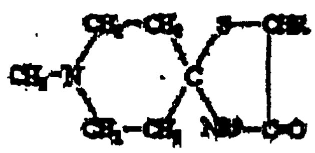

wo R Ethyl ist und $R^0$ Wasserstoff ist.

**14.** Verbindung AF267, welche eine Verbindung der Formel (K) ist

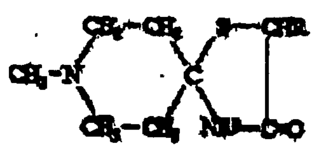

wo R Ethyl ist und $R^0$ Wasserstoff ist.

**15.** Verbindung nach Anspruch 14, welche das Hydrochloridsalz von AF267 ist.

**16.** Verbindung AF266, welche eine Verbindung der Formel (K) ist:

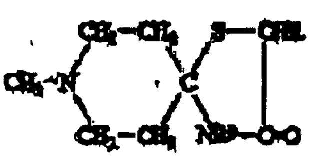

wo R Methyl ist und R° Methyl ist

**17.** Verbindung AF272, welche eine Verbindung der Formel (K) ist:

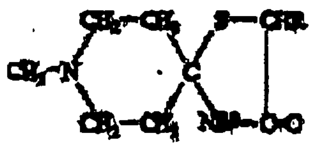

wo R Ethyl ist und R° Methyl ist.

**18.** Verbindung AF261, welche eine Verbindung der Formel (K) ist:

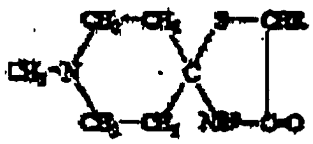

wo R Methyl ist und $R^0$ Wasserstoff ist.

**19.** Pharmazeutische Zusammensetzung, umfassend ein Enantiomer nach einem der Ansprüche 10 bis 13 oder eine Verbindung nach einem der Ansprüche 14 bis 18 zusammen mit einem pharmazeutisch verträglichen Verdünnungsmittel, Träger oder Hilfsstoff.

**20.** Pharmazeutische Zusammensetzung nach Anspruch 19 zusammen mit mindestens einer weiteren pharmakologisch wirksamen Verbindung, ausgewählt aus der Gruppe, bestehend aus Physostigmin, Tetrahydroaminoacridin, Cholin, Lecithin, Piracetam, Aniracetam, Pramiracetam, Oxiracetam, 4-Aminopyridin, 3,4-Diaminopyridin, Somatostatin, Pirenzepin, N-Methylatropin, N-Butylscopolamin, Scopolamin, Clonidin, Quanfamicin, Propanthelin, Methanthelin, Glycopyrrolat, Tropenzilium, Nortriptylin, Amitriptylin, Imipramin, Minaprin, Secoverin, AFDX-116, Nicotin, Alaproclat, Zimelidin, Deprenyl und Nervenwachstumsfaktor.

**21.** Pharmazeutische Zusammensetzung nach Anspruch 19 oder 20, welche in einer für orale, rektale, parenterale oder transdermale Verabreichung oder für Verabreichung durch Insufflation oder Nasenspray geeigneten Form ist und im Fall von transdermaler Verabreichung die Zusammensetzung als zusätzliche Komponente eine Fettsäure mit niedrigem Molekulargewicht umfassen kann.

**22.** Pharmazeutische Zusammensetzung nach Anspruch 19 zur Verwendung bei der Behandlung von Krankheiten des zentralen und peripheren Nervensystems bei Säugetieren, welche Zusammensetzung eine Menge, wirksam zur Verwendung bei der Behandlung der Krankheiten, von mindestens einer Verbindung, wie in einem der Ansprüche 1 bis 16 definiert, zusammen mit mindestens einem pharmazeutisch verträglichen Verdünnungsmittel, Träger oder Hilfsstoff, und als weitere pharmakologisch wirksame Verbindung Nervenwachsturnsfaktor umfaßt, mit der Maßgabe, daß die mindestens eine Verbindung in einer Menge vorhanden ist, welche die Nervenwachstumsaktivität des Nervenwachstumsfaktors begünstigt.

## Revendications

**1.** Utilisation d'un composé de la formule (1) dans la préparation d'un médicament pour une utilisation dans le traitement d'une maladie du système nerveux central ou périphérique chez un mammifère, la formule (I) étant;

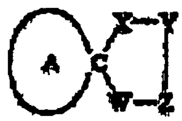

dans laquelle X est >S, Y est >CRR', Z est >C=O et W est>NR$^0$;

dans laquelle R est choisi parmi H, un groupe alkyle $C_{1-6}$, alkoxy $C_{1-6}$, hydroxyalkyle $C_{2-6}$, alcényle $C_{2-6}$, alcynyle $C_{2-6}$, phényle et alkyle $C_{1-6}$ substitué par 1, 2 ou 3 phényles;
R' est choisi parmi H, un groupe alkyle $C_{1-6}$, alkoxy $C_{1-6}$, hydroxyalkyle $C_{2-6}$, alcényle $C_{2-6}$, alcynyle $C_{2-6}$, phényle et alkyle $C_{1-6}$ substitué par 1, 2 ou 3 phényles; et
$R^0$ est choisi parmi H, un groupe alkyle $C_{1-6}$, alkoxy $C_{1-6}$, hydroxyalkyle $C_{2-6}$, alcényle $C_{2-6}$, alcynyle $C_{2-6}$, phényle et alkyle $C_{1-6}$ substitué par 1, 2 ou 3 phényles et alcanoyle $C_{1-6}$; et
A est un cycle (M):

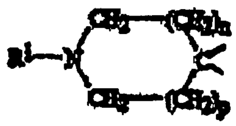

qui est non substitué ou qui est substitué par 1-3 substituants choisis parmi un groupe alkyle $C_{1-6}$ et hydroxyle et dans lequel n et p sont chacun 0, 1, 2 ou 3, sous réserve que n + p est 1-3; et $R^1$ est choisi parmi H, un groupe alkyle $C_{1-6}$, alcényle $C_{2-6}$, alcynyle $C_{2-6}$, cycloalkyle $C_{3-7}$, alkyle $C_{1-6}$ substitué par 1-6 atomes d'halogène, hydroxy-$C_{1-6}$-alkyle, alkoxy $C_{1-6}$, alkylthio $C_{1-6}$, $C_{1-6}$-alkoxy-$C_{1-6}$-alkyle, carboxy-$C_{1-6}$-alkyle, ($C_{1-6}$-alkoxy) cabonyl-$C_{1-6}$-alkyle, amino-$C_{1-6}$-alkyle, mono-($C_{1-6}$-alkyl)amino-$C_{1-6}$-alkyle, di-($C_{1-6}$-alkyl)amino-$C_{1-6}$-alkyle, 2-oxopyrrolidin-1-ylméthyle, aryle, diarylméthylol, alkyle $C_{1-6}$ substitué par un ou deux groupes aryles, $C_{1-6}$-alcanoyle et arylcarbonyle, où aryle indique un phényle non substitué ou un phényle substitué par 1-3 substituants choisis parmi un halogène, un groupe alkyle $C_{1-6}$, alkoxy $C_{1-6}$ et $CF_3$;
incluant des sels pharmaceutiquement acceptables, des composés quaternaires dérivés structurellement des composés au niveau d'un atome d'azote tertiaire, des énantiomères et des racémates de ceux-ci.

2. Utilisation d'un composé suivant la revendication 1, pour une utilisation dans la préparation d'un médicament présentant une activité biologique choisie parmi (a) une activité d'agoniste muscarinique, (b) une activité de type neurotrophique ou synergique avec NGF, (c) une activité de sécrétion d'une protéine précurseur d'amyloïde (APP) et une activité diminuant les β-amyloïdes, (d) une activité augmentant la proportion de protéines τ déphospborylées et (e) une activité de type NGF.

3. Utilisation suivant la revendication 1, dans laquelle le composé de la formule (I) est de la formule (K):

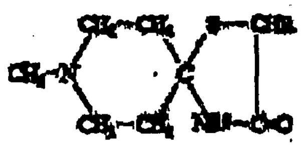

dans laquelle R est choisi parmi H, un groupe alkyle $C_{1-6}$, alkoxy $C_{1-6}$, hydroxyalkyle $C_{2-6}$, alcényle $C_{2-6}$, alcynyle $C_{2-6}$, phényle et alkyle $C_{1-6}$ substitué par 1, 2 ou 3 phényles; et

$R^0$ est choisi parmi H, un groupe alkyle $C_{1-6}$, alkoxy $C_{1-6}$, hydroxyalkyle $C_{2-6}$, alcényle $C_{2-6}$, alcynyle $C_{2-6}$, phényle et alkyle $C_{1-6}$ substitué par 1, 2 ou 3 phényles et alcanoyle $C_{1-6}$;
incluant des sels pharmaceutiquement acceptables, des composés quaternaires dérivés structurellement des composés au niveau d'un atome d'azote tertiaire, des énantiomères et des racémates de ceux-ci.

4. Utilisation suivant la revendication 3, dans laquelle le composé de la formule (K) est AF267 où R est un groupe

éthyle et $R^0$ est un hydrogène.

**5.** Utilisation suivant la revendication 4; dans laquelle le composé de la formule (K) est le sel de chlorhydrate de AF267.

**6.** Utilisation suivant la revendication 3, dans laquelle le composé de la formule (K) est AF266 où R est un groupe méthyle et $R^0$ est un groupe méthyle.

**7.** Utilisation suivant la revendication 3, dans laquelle le composé de la formule (K) est AF272 où R est un groupe éthyle et $R^0$ est un groupe méthyle.

**8.** Utilisation suivant l'une quelconque des revendications précédentes, dans laquelle le composé de la formule (I) est sous la forme d'un énantiomère.

**9.** Utilisation suivant la revendication 7, lorsqu'elle est dépendante de la revendication 4, dans laquelle le composé de la formule (I) est AF267B, qui est (-)AF267.

**10.** Enantiomère d'un composé de la formule (I):

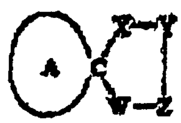

dans laquelle X est >S, Y est >CRR', Z est >C=O et W est >$NR^0$;

dans laquelle R est choisi parmi H, un groupe alkyle $C_{1-6}$, alkoxy $C_{1-6}$, hydroxyalkyle $C_{2-6}$, alcényle $C_{2-6}$, alcynyle $C_{2-6}$, phényle et alkyle $C_{1-6}$ substitué par 1, 2 ou 3 phényles;
R' est choisi parmi H, un groupe alkyle $C_{1-6}$, alkoxy $C_{1-6}$, hydroxyalkyle $C_{2-6}$, alcényle $C_{2-6}$, alcynyle $C_{2-6}$, phényle et alkyle $C_{1-6}$ substitué par 1, 2 ou 3 phényles; et
$R^0$ est choisi parmi H, un groupe alkyle $C_{1-6}$, alkoxy $C_{1-6}$, hydroxyalkyle $C_{2-6}$, alcényle $C_{2-6}$, alcynyle $C_{2-6}$, phényle et allcyle $C_{1-6}$ substitué par 1, 2 ou 3 phényles et alcanoyle $C_{1-6}$ ; et
A est un cycle (M):

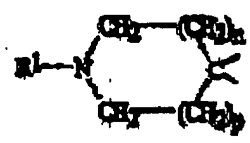

dans lequel n et p sont chacun 0, 1, 2 ou 3, sous réserve que n + p est 1-3; et
$R^1$ est choisi parmi H, un groupe alkyle $C_{1-6}$, alcényle $C_{2-6}$, alcynyle $C_{2-6}$, cycloalkyle $C_{3-7}$, alkyle $C_{1-6}$ substitué par 1-6 atomes d'halogène, hydroxy-$C_{1-6}$-alkyle, alkoxy $C_{1-6}$, alkylthio $C_{1-6}$, $C_{1-6}$-alkoxy-$C_{1-6}$-alkyle, carboxy-$C_{1-6}$-alkyle, ($C_{1-6}$-alkoxy)carbonyl-$C_{1-6}$-alkyle, amino-$C_{1-6}$-alkyle, mono-($C_{1-6}$-alkyl)amino-$C_{1-6}$-alkyle, di-($C_{1-6}$-alkyl)amino-$C_{1-6}$-alkyle, 2-oxopyrrolidin-1-ylméthyle, aryle, diarylméthylol, alkyle $C_{1-6}$ substitué par un ou deux groupes aryles, alcanoyle $C_{1-6}$ et arylcarbonyle, où aryle indique un phényle non substitué ou un phényle substitué par 1-3 substituants choisis parmi un halogène, un groupe alkyle $C_{1-6}$, alkoxy $C_{1-6}$ et $CF_3$; incluant des sels pharmaceutiquement acceptables et des composés quaternaires dérivés structurellement des composés au niveau d'un atome d'azote tertiaire.

**11.** Enantiomère suivant la revendication 10, pour une utilisation dans un procédé de traitement du corps d'un humain ou d'un animal par thérapie.

**12.** Enantiomère suivant la revendication 10, pour une utilisation dans le traitement d'une maladie du système nerveux central ou périphérique chez un mammifère.

**13.** Enantiomère suivant la revendication 12, qui est AF267B, c'est-à-dire (-)AF267, qui est un composé de la formule (K):

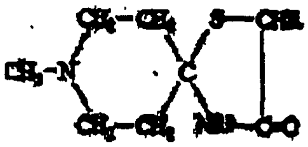

dans laquelle R est un groupe éthyle et R° est un hydrogène.

**14.** Composé AF267 qui est un composé de la formule (K):

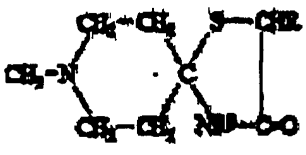

dans laquelle R est un groupe éthyle et $R^0$ est un hydrogène.

**15.** Composé suivant la revendication 14, qui est le sel de chlorhydrate de AF267.

**16.** Composé AF266 qui est un composé de la formule (K):

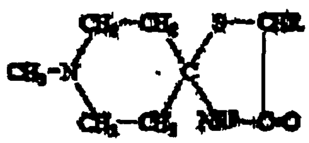

dans laquelle R est un groupe méthyle et $R^0$ est un groupe méthyle.

**17.** Composé AF272 qui est un composé de la formule (K):

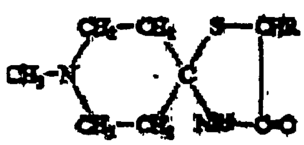

dans laquelle R est un groupe éthyle et $R^0$ est un groupe méthyle.

**18.** Composé AF261 qui est un composé de la formule (K):

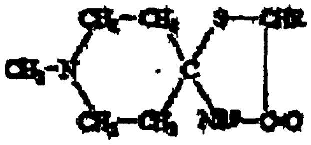

dans laquelle R est un groupe méthyle et $R^0$ est un hydrogène.

**19.** Composition pharmaceutique comprenant un énantiomère suivant l'une quelconque des revendications 10 à 13, ou un composé suivant l'une quelconque des revendications 14 à 18, accompagné d'un diluant, d'un excipient ou d'un adjuvant pharmaceutiquement acceptable.

**20.** Composition pharmaceutique suivant la revendication 19, accompagnée d'au moins un composé pharmacologiquement actif supplémentaire choisi dans le groupe constitué de physostigmine, de tétrahydroaminoacridine, de choline, de lécithine, de piracétam, d'aniracétam, de pramiracétam, d'oxiracétam, de 4-aminopyridine, de 3,4-diaminopyridine, de somatostatine, de pirenzépine, de N-méthylatropine, de N-butylscopolamine, de scopolamine, de clonidine, de quanfamicine, de propanthéline, de méthanthéline, de glycopyrrolate, de tropenzilium, de nortriptyline, d'amitriptyline, d'imipramine, de minaprine, de sécovérine, de AFDX-116, de nicotine, d'alaproclate, de zimélidine, de déprényle et du facteur de croissance des cellules nerveuses (NGF).

**21.** Composition pharmaceutique suivant la revendication 19 ou 20, qui se trouve sous une forme appropriée pour une administration orale, rectale, parentérale ou transdermique, ou pour une administration par insufflation ou par spray nasal, et dans le cas d'une administration transdermique, la composition peut comprendre, comme composant supplémentaire, un acide gras à bas poids moléculaire.

**22.** Composition pharmaceutique suivant la revendication 19, pour une utilisation dans le traitement de maladies du système nerveux central et périphérique chez des mammifères, laquelle composition comprend une quantité efficace pour une utilisation dans le traitement desdites maladies d'au moins un composé suivant l'une quelconque des revendications 1 à 16, accompagné d'au moins un diluant, un excipient ou un adjuvant pharmaceutiquement acceptable, et comme composé pharmacologiquement actif supplémentaire, le facteur de croissance des cellules nerveuses, sous réserve que ledit au moins un composé soit présent dans une quantité qui favorise l'activité de croissance des cellules nerveuses du facteur de croissance des cellules nerveuses.